# EUROPEAN PATENT APPLICATION

(11) **EP 2 108 959 A1**
(43) Date of publication of application: **14.10.2009**
(21) Application number: 09163806.4
(22) Date of filing: 14.09.2006
(51) Int. Cl.: G01N 33/68, C12Q 1/68, G01N 33/50, C07K 14/825

(54) **In vitro assay for identification of allergenic proteins**

(30) Priority: 16.09.2005 SE 0502047; 27.09.2005 US 596473 P
(62) Divisional of application: 06784249.2
(71) Applicant: Biovator Technologies AB, 103 95 Stockholm (SE)
(72) Inventor: Lundgren, Hanna, 151 33 Södertälje (SE); Cederbrant, Karin, 146 38 Tullinge (SE); Thorell, Jan, 183 68 Täby (SE)
(74) Representative: Rystedt, Per Hampus

(57) **Abstract**

The present invention relates to a process for in vitro evaluation of a potentially allergenic or tissue irritating substance whereby test cells are cultivated in the presence of the substance, and the presence of up regulated genes chosen from MT1G, MT1B;MT1A, MT1E, MT1F, MT1H, or expression products from them are measured. The invention also regards use of the expression products from one or more of the genes for in vitro analysis of allergy or tissue irritation.

It also relates to a probe comprising at least three nucleic acids, preferably 3 - 40, especially 5 - 15 chosen from RNA complementary to the RNA corresponding to any of the genes and the use thereof for in vitro analysis of allergy or tissue irritation.

Further it regards a reagent kit comprising one or more probes that recognize products produced during the expression of any the genes.

## Description

The present invention relates to a process for in vitro evaluation of a potentially allergenic or tissue irritating substance whereby test cells are cultivated in the presence of the substance, and the presence of up certain regulated genes stated in claim 1 or expression products from them are measured. This method is called gene activation profile assay, GAPA.The invention also regards use of the expression products from one or more of the genes for in vitro analysis of allergy or tissue irritation.

It also relates to a probe comprising at least three nucleic acids, preferably 3 - 40, especially 5 - 15 chosen from RNA complementary to the RNA corresponding to any of the genes and the use thereof for in vitro analysis of allergy or tissue irritation.

Further it regards a reagent kit comprising one or more probes that recognize products produced during the expression of any the genes.

### Prior art.

Today there is no validated and reliable *in vitro* test available to predict the allergic response towards chemical entities. The tests used today are *in vivo* animal tests and on the account of ethical aspects there is a great demand of finding an *in vitro* method that can replace the currently used animal tests. Allergic reactions can be really serious for the person affected so there is a great demand from e.g. the pharmaceutical-, cosmetic- and the food industry to be able to identify these substances in an as early phase as possible.

Previous studies have shown that neopterin and interleukin-8 (IL-8), produced by blood cells, may be reliable signal molecules to identify allergenic substances¹. This hypothesis that lead to a Swedish patent (No. 506 533, WO 97/16732) directed to an in vitro method for the identification of human allergens and T-lymphocyte antigens. The method covered by this patent was named cytokine profile assay (CPA). The concept of this test is that allergenic substances are able to induce specific patterns of neopterin and IL-8 production, measured in the supernatant of cultivated human peripheral blood mononuclear cells (PBMC). Further validation studies of the CPA lead to the preferable use of a human monocyte cell-line as a reference system. Also, the method appeared most suitable to identify proteins known to induce type I allergy.

### Allergen

Antigens able to stimulate hypersensitivity mediated by an immunologic mechanism are referred to as allergens. Allergens induce a cellular or humoral response in the same way as any other antigen, generating activated T-cells, antibody-secreting plasma cells and subsequently memory cells.

A lot of effort has been done to identify a common chemical property of an antigen, but it has all failed because of the complexity of the immune system.

The chemical nature of allergens

### Proteins

Proteins have the ability to induce an allergic response in susceptible individuals. The reaction requires complex interactions between the protein and the immune system, which are notoriously difficult to predict. Known allergenic proteins normally have a molecular weight between 15000 and 40000² and they are often associated with allergy to environmental factors such as animal dander, enzymes, pollen and foods giving an allergenic reaction of type I.

To be defined as allergenic, proteins have to contain epitopes detectable by immunoglobulin E and T-cells but it is considered that other features and characteristics of proteins give them their overall allergenicity. Important factors that contribute to the likelihood of food proteins to induce an allergic response are exposure time and stability. For example known food allergens are shown to be stable in the gastric model, representing the gastrointestinal tract, used by Astwood et al.³ compared to the more fastly digested non-allergenic proteins. The rationale for this is that stable proteins persist long enough time in the gastrointestinal tract in its intact form to provoke an immune response.

Another characteristic property is post-translational glycosylation that have been observed happening to many allergens⁴ raising the possibility that the glycosyl groups may contribute to their allergenicity. The glycosylation influence the physical properties of the protein, including altered stability, solubility, hydrophobicity and electrical charge, and hence alter its allergenic properties, perhaps by increasing uptake and consequently detection of the protein by the immune system. Enzymatic activity can also be correlated to allergenicity. For example, introduction of enzymes into detergents can make the detergent able to cause allergic sensitization⁵.

Many allergens share some homology and the primary sequence of a protein can therefore, at least in part, be associated with allergenic properties. On the other hand, when the actual allergenic epitope is considered (approximately 10-15 amino acid long) no general homology for allergenic amino acid sequence emerges. Studies have also showed that allergenic proteins; tend to be ovoid in shape, have repetitive motifs, are heat stable, and that the proteins disulfide bounds contribute to the allergenicity⁶.

In summary many factors can contribute to the allergenicity of a protein, either independently or in concert:
- size and structure
- presence of T- and B-cell epitopes able to induce a immunologic response
- resistance to heat and degradation
- glycosylation status
- biological function (in particular if enzymatic activity is present)

### Haptens

Low-molecular-weight chemicals, for instance isocyanates, can also behave as allergens and they are called haptens. These molecules generally have a molecular weight below 700. Haptens are antigenic but not immunogenic meaning that they cannot by them selves induce an immune response. However, when they are coupled to a large protein, i.e. soluble or cell-bound host proteins so called carrier protein, it forms an immunogenic hapten-carrier conjugate. The sensitization capacity of a hapten allergen depends on its ability to form these hapten-protein complexes. The interaction between hapten and protein involves, in the vast majority of cases, a covalent, and therefore irreversible, bound. This implies that the hapten has a chemical reactivity characteristic that allows it to form bonds with the side-chains of amino acids. Frequent targets are cysteine, histidine and lysine, depending on the structure of the hapten. The sensitizer acts as an electrophil and the protein acts as a nucleophil in most of these reactions with the nucleophilic function in the side groups (-NH2, -SH, -S, -N, -NH and -OH) of the amino acids. Metals on the other hand can form coordination bonds with proteins. Some haptens may instead easily form free radicals, which also bind to proteins using a free radical mechanism⁷.

According to the classical model by Landsteiner a hapten entering the body, chemically linked to a carrier protein, generates antibodies specific to: the hapten determinant, epitopes on the carrier protein and new epitopes, formed by the conjugate of hapten and carrier. However, it has also been shown that a hapten alone, without binding to a carrier protein, is able to induce a T-cell response. Hapten-specific T cells recognize hapten-modified MHC-peptide complexes, suggesting that the hapten modifies the structure of the MHC molecules, the bound peptide, or both, and that it is the modified structure that is recognized by the T cells⁸.

Haptens normally induce a hypersensitivity reaction of type IV resulting in skin contact allergy; an important property of many haptens is therefore the ability to penetrate the skin barrier. Many different xenobiotics such as drugs, metals, and chemicals, but also peptide hormones, and steroid hormones, can function as haptens, giving a type IV hypersensitivity reaction.

Haptens may vary from simple metal ions to complex aromates. Common properties among haptens are:
- low-molecular-weight
- ability to penetrate the skin barrier
- chemical reactivity characteristics that allows it to form bonds with the side chains of amino acids or properties able to modify the structure of the MHC molecules and/or the bound peptide

### Presentation of allergen by APC - generation of an allergic response

The antigen-presenting cells (APC) are the key players in the generation of an allergen-specific immune response.

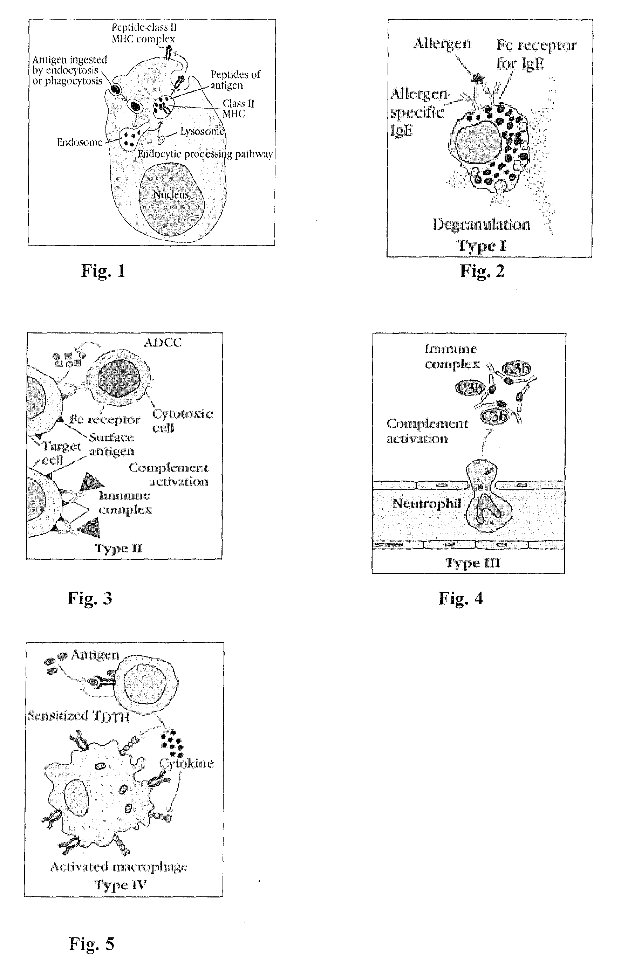

APCs, includes macrophages, B lymphocytes and dendritic cells, have two characteristics: they express class II MHC molecules on their membranes and they are able to stimulate T-cells activation. In order to be recognized by the immune system all antigens entering the body have to be processed and presented. Exogenous antigens, like protein allergens, enter the cells either by endocytosis or phagocytosis of APCs, followed by degradation into peptide fragments and subsequent presentation of antigenic structures by class II molecules on the cell surface, Figure 1. In this way possible antigenic structures gets presented to T-lymphocytes on the APC surface. T lymphocytes carry unique antigen-binding molecules on their APC surface, called T-cells receptors. These are able to recognize antigenic structures of the size 9-15 amino acids. When the T-cell finds an APC presenting a peptide matching its receptors it gets activated and secretes cytokines that contribute to activation of B-cells, T-cells and other cells. Simultaneously a B-cell, with antibodies recognizing the same antigen, interacts with the antigen, gets activated by the T-cell and differentiates into antibody-secreting plasma cells and memory cells. Antibodies, as well as T-cells are central actors in the elicitation of an allergic reaction.

### Allergy

Allergy, a hypersensitivity reaction initiated by immunologic mechanisms, is the result of adverse immune responses against, for example, common substances derived from plants, foods or animals. Different immune mechanisms can give rise to hypersensitivity reactions and therefore P.G.H. Gell and R.R.A. Coombs suggested in 1968 a classification scheme where hypersensitivity reactions are divided into four groups. Each group involves various mechanisms, cells, and mediator molecules, and it is important to keep in mind that the mechanisms are complex and the boundaries between categories are blurred. Three of the four types are mediated by antibody or antigen-antibody complexes and consequently occur within the humoral branch, the fourth type occur within the cell-mediated branch of the immune system.

| | |
|---|---|
| Type I: | IgE antibody mediated |
| Type II: | Antibody-mediated (IgG or IgM antibody mediated) |
| Type III: | Immune complex mediated (IgG or IgM antibody mediated) |
| Type IV: | Delayed type hypersensitivity (DTH), cell mediated |

Characteristic for a hypersensitivity reaction is the reproducibility; T- and B-cells will form allergen specific memory cells able to give a response whenever exposed to the allergen.

### Type I hypersensitivity

The principle of type I hypersensitivity is based on antibody production to an allergen using the same mechanism as a normal humoral response performs when meeting an antigen. The distinction is that during a type I hypersensitivity reaction IgE instead of IgG antibodies are secreted by the plasma cells.

Upon exposure to a type I allergen, B-cells get activated and develop into IgE-secreting plasma cells and memory cells. When Ig E binds to mast cells and blood basophiles these cells release pharmacologically active mediators, Figure 2, causing smooth muscle contraction, increased vascular permeability and vasodilation.

In the normal immune response, IgE antibodies are produced as a defense against parasitic infections but when they are produced as a response to an allergen the person is said to be atopic. Johansson et al⁹defines atopy as "a personal or familial tendency to produce IgE antibodies in response to low doses of allergens, usually proteins, and to develop typical symptoms such as asthma, rhinoconjunctivitis, or eczemal/dermatitis". This reaction can occur after exposure to common environmental antigens for instance nuts and wasp venom.

The reaction is partly hereditary and occurs 5-20 minutes after exposure and can if untreated lead to death. Thus, type I hypersensitivity is regarded as the most serious hypersensitivity reaction¹⁰.

### Type II hypersensitivity

This is an antibody-mediated cytotoxic hypersensitivity reaction and it involves IgG/IgM-mediated destruction of cells. Type II hypersensitivity can occur through antibodies activating the complementary system to create pores in the membrane of the target cell, which leads to cell death. Cell destruction can also occur by antibody-dependent cell-mediated cytotoxicity (ADCC). Antibodies are formed against antigen on the cell surface. After they attach to the surface cytotoxic cells bind to the antibody. This promotes destruction of the target cell, Figure 3.

Transfusion reaction and erythroblastosis fetalis are example of type II hypersensitivity reactions. It takes around five to eight hours between exposure to antigen and clinical reaction¹⁰_{.}

### Type III hypersensitivity

In these reaction IgG/IgM antibodies, bound to antigen, together generate an immune complex. These immune complexes generally facilitate the clearance of antigen but if antigen is in excess many small immune complexes are generated that are not easily cleared by phagocytic cells, Figure 4. This can lead to type III hypersensitive tissue damaging expressed as an inflammatory reaction.

A type III hypersensitivity reaction can be observed in autoimmune diseases (e.g. rheumatoid arthritis), drug reactions (e.g. allergies to penicillin) and infectious diseases (e.g. malaria). The reaction occurs between 4 and 8 hours after exposure¹⁰.

### Type IV hypersensitivity

This reaction is also referred to as delayed hypersensitivity and may develop as a result of skin exposure to low molecular weight chemical substances (hapten) leading to allergic contact dermatitis. The mechanism of type IV hypersensitivity is characterized by the formation of allergen-specific T-cells. No antibodies are involved in this reaction.

When T cells get activated, they secret cytokines, leading to activation of an influx of nonspecific inflammatory cells, where macrophages are major participants, resulting in a local inflammation (an eczema), Figure 5. In the normal immune response this reaction plays an important role in host defense against intracellular pathogens.

Antigens typically giving rise to a delayed hypersensitivity may be synthetic or naturally occurring substances, such as drugs, metals or plant components. The delayed hypersensitivity reaction gets noticeable 24-48 hours after contact with the allergen resulting in an inflammatory reaction in the skin at the site of exposure¹⁰.

### Irritant reaction

There are other forms of hypersensitivity than the allergic types. Reaction after exposure to an irritant is an example of non-allergic hypersensitivity. A characteristic of this response is release of pro-inflammatory mediators, for example the cytokines tumor necrosis factor α (TNFα) and interleukin 6 (IL6)¹¹. The reaction is similar to a type IV hypersensitivity reaction but the main difference is that this process does not require sensitization and therefore no memory T-cells develop like in a type IV reaction¹². Antigen-specific antibodies are neither present. An irritant reaction can occur as a response after; a single contact with a powerful irritant, such as benzalkonium chloride, frequent work in a wet environment, or frequent contact with a weak irritant chemical. Irritancy has been shown to have a profound effect on the dynamics of contact allergen sensitization¹², meaning that allergic contact dermatitis occur more often if an irritant is present together with the antigen.

### Predictive test methods

During the years several predictive tests for identification of possible allergenic potential of chemicals and proteins have been used. Both human and animals have served as test subjects. Test methods using humans were mainly developed between 1944 and 1980. A great disadvantage of these tests is that many volunteers are needed to make the test results reliable. There is also a risk that the volunteers become sensitized for the rest of their lives and develop eczema to the test chemicals upon future exposures. Since this is a great ethical problem no tests are performed on humans today.

Animal tests to identify contact sensitizers have been available for many years. They are all *in vivo* methods and the most commonly used to identify skin sensitizers are the guinea pig maximization test (GPMT) and the Buehler test, an occluded patch test in guinea pigs without adjuvant. Another evaluated and accepted test used to identify skin sensitizers is the Local Lymph Node Assay (LLNA).

### Guidelines

To get a standardized system for Europe and the world to evaluate new drugs and other products on the global market a system with various organizations evaluating new methods has been unfold.

The Organization for Economic Cooperation and Development (OECD) is an organization that groups 30 member countries sharing a commitment to democratic government and the market economy. The organization also has an active relationship with some 70 other countries and organizations, giving a global reach. The organization produces internationally agreed instruments, decisions and recommendations to promote rules of the game in areas where multilateral agreement is necessary for individual countries to make progress in a globalised economy.

In the 406 Test Guideline (adopted in 1981) for OECD the GPMT and the Buehler test were recommended for the assessment of allergic contact dermatitis chemicals. These two tests have been used until recently. In April 2002 LLNA was incorporated into a new test guideline (No. 429; Skin Sensitization: Local Lymph Node Assay) by the OECD, adopted in July same year. In parallel, the European Union has prepared a new test guideline for the assay. The LLNA is also recommended by the most recent Food and Drug Administration (FDA) guideline on immunotoxicity¹³ where suggested to be advantageous over the guinea pig assays. Interagency Coordinating Committee on the Validation of Alternative Methods (ICCVAM) concludes that LLNA offers important animal welfare benefits with respect to both reduction and refinement¹⁴.

### Alternative methods

The present animal based tests are time consuming, expensive to carry through and include many ethical aspects since animals are used. Because of this a lot of research has been done and some new methods have been developed to identify substances with allergenic properties.

### In vivo/in vitro

Dearman et al.^{15;16} have tried to develop a method to predict the allergenic potential of chemical allergens by measuring levels of different cytokines from lymph node cells. In mice, topically exposed to the respiratory allergen touene diisocyanate (TDI) and the skin sensitizer dinitrofluorobenzene (DNFB), they monitored changes in cytokine levels of interferon γ (IFN-γ), IL-4 and IL-10. The data presented suggest that relative cytokine secretion patterns induced in the draining lymph node cells of mice may characterize different classes of chemical allergens, but the method has to be further evaluated.

Since type IV reactions involve both antigen presenting cells (APC) and T-cells a culture system containing both stimulatory APC and responding T-cells would appear to provide the best approach for the development of an *in vitro* test predicting allergenic properties of a chemical. Several attempts have been made to establish such an *in vitro* system however without success. The principal APC in the skin is consider to be the langerhans cell (LC) and therefore several investigators have focused on events that occur in LC following exposure to chemical haptens and irritants. Many techniques have been developed to isolate populations of LC from human and murine sources to enable an establishment of an *in vitro* method mimicking the course of events occurring in the skin when exposed to a type IV allergen. To date no LC line has been established and therefore the number of cells has been the limiting factor in the development of LC-based *in vitro* methods.

The EpiDerm model is a method able to detect the irritative potential of a substance as evaluated by the European Centre for the Validation of Alternative Methods (ECVAM). The experimental procedure consists of normal, human-derived epidermal keratinocytes, which have been cultured to form a multi-layered, highly differentiated model of the human epidermis. The tissue is transferred to a plate, containing medium and the substance is applied on top of the tissue. Cell viability is calculated for each tissue as a percentage of the negative control tissue. The test substance is classified according to remaining cell viability following exposure of the test substance. Theory for the test is founded on the knowledge that irritating chemicals show cytotoxicity following short-term exposure to epidermis ^{17;18}. However, this model has not been used for classification of possible allergens.

### In silico

In parallel to the biological studies another approach has become more and more important, namely the study of structure-activity relationships (SARs). With this method molecular or physicochemical properties of known molecules are used to predict the allergenic potential of unknown substances. Structure, physicochemical and electronic data for a new compound are compared with data on chemical structures known to inherit sensitization risks. The final use of a system of this type is to answer questions like: which compound may or may not be sensitizing.

DEREK (Deductive Estimation of Risk from Existing Knowledge) is a database based on this principle. The system consist of a "control" program that analyses the structure of the molecules and a database consisting of "rules" in the form of substructures known to be associated with allergenic properties. DEREK then estimates the "risk" for the compound to be allergenic. A limitation of this system is that the program does not take into consideration metabolization of the substance, a circumstance that is important for allergens. The process is based simply on the structure of the tested molecule, which is not necessarily that which, for example in type IV allergy, reacts with the skin proteins. Another approach is to create databases only containing experimental and case information. Examples of such a data base is that developed in Palo-Alto by CCS Associates in collaboration with H.I. Maibach of the University of San Francisco and Professor C. Benzra¹⁹ where the main sources of data used are the case of allergy published in Contact Dermatitis since 1975. The limitation of this system results from the way reference data were compiled. The data is based on historical material, newer substances are not included. Another problem is that the stored data is based on scientific publications where a severe reaction in a few patients is often better documented than moderate reactions in a large number of patients, resulting in that moderate but common reactions can fail to be detected. Other databases with only allergenic substances are Allergome and Allermatch. It is also possible to compare sequences through the database SWISS-PROT, having 92,000 annotated protein sequences and is cross-referenced with approximately 30 other databases.

### Current requirements for new tests

The primary limitation of the already validated and accepted tests is that they are only able to detect type IV allergens, inducing contact allergy. Accordingly, there is today no validated and accepted test which can identify an unknown substance causing an allergic reaction of type I, a reaction with a fast course of event and often dismal prospect¹⁰. Opportunities for the development of alternative tests to detect allergic reactions *in vitro* are great due to increased requirements from the society and a lot of effort has been put into this area. There is optimism in that the new technologies that are emerging, or which are already available, will provide realistic opportunities for the design of alternative approaches. Continued development of our understanding of the chemical and biological aspects of allergic reactions and with the application of genomics/proteomics to this field may in the future permit the replacement of animal methods.

### New test methods - criteria of acceptance

To get a new *in vitro* test accepted and ready for the market a procedure aiming at establish relevance and reliability is required according to The European Agency for the Evaluation of Medicinal Products committee for proprietary medicinal products (CPMP).

### Phase I: Test development and definition

The test has to have a defined objective and the laboratory behind the project has to describe the operating procedures thoroughly, to make it possibly for other laboratories to reproduce the test. Specificity, sensitivity and reproducibility, of the test, must be related to supplied data. A conclusive number of reference substances including positive and negative controls must be tested to establish the tests consistency.

### Phase II: Test optimization

A multi-center study, involving laboratories from different countries, has to be made to assess the test. The tests utility, reliability, robustness and practice ability must be described, emphasized the technical improvement of the test compared to the original method. In this study the contributory laboratories have to define and evaluate a limited and conclusive number of reference substances, including positive and negative controls. It is essential that the multi-center study is published in an international peer reviewed scientific journal.

### Phase III: Validation

The test has after phase I and II its final configuration and an multi-center study with a large number of laboratories from different counties has to be done. The aim is to compare the relevance of the proposed test to the accepted standard *in vivo* method. An increased number of appropriate chosen relevant products are tested. Also this study has to be published.

### Phase IV: Setting-up or taking part in an international data bank

To create an international data bank is necessary to improve knowledge of the performance of the test, especially if the test should be performed on a routine basis.

During the development of the GAPA test the variations between test results was initially still large since the cell source was taken from different individuals. To make the test more stable, reproducible, and commercially practicable a more standardized cell source was looked for.

A screening of 13 the monocyte/macrophage cell lines took place. Three substances with known allergenicity and irritancy were used. The outcome of the screening resulted in that the cell line MonoMac-6 was found suitable for the GAPA-test.

### Mono Mac 6

The parent cell line, Mono Mac, was established from the peripheral blood of a 64-year-old male patient diagnosed in 1985 with relapsed acute monoblastic leukemia (AML FAB M5) following myeloid metaplasia. The blood sample, from which the parent cell line was established, was taken one month before the patient's death. This gave rise to two subclones, Mono Mac 1 and Mono Mac 6, and they both were assigned to the monocyte lineage on the basis of morphological, cytochemical and immunological criteria. Mono Mac 6 appears to constitutively express phenotypic and functional features of mature monocytes²⁰.

Mono Mac 6 grows in suspension as single round/multiformed cells or small in clusters, sometimes loosely adherent. They have a doubling time of about 60 hours when incubated at 37°C with 5% CO₂ and a maximal density at about 1.0 x 10⁶ cells/ml. The cells have a diameter of approximately 16µ, with a round or intended nucleus with sometimes one or two nucleoli as verified by light microscopy. In 4.8 ±1,9% of the cells 2-4 nuclei are observed. The cytoplasm contains many mitochondria, numerous rough endoplasmatic reticulum cysternae, a prominent Golgi complex, lysosomes, coated vesicles, endocytic vesicles and multivesicular bodies. Mono Mac 6 has the ability to readily phagocytose antibody-coated erythrocytes, proving Mono Mac 6 to bee representative of mature monocytes²¹.

The inventors have found that certain genes are up regulated when allergenic or tissue irritating substances are present. Their expression products may be measured as an indication of the substances.

### Summary of the invention

The present invention relates to a process for in vitro evaluation of a potentially allergenic or tissue irritating substance whereby test cells are cultivated in the presence of the substance, and the presence of up certain regulated genes stated in claim 1 or expression products from them are measured. The invention also regards use of the expression products from one or more of the genes for in vitro analysis of allergy or tissue irritation.

It also relates to a probe comprising at least three nucleic acids, preferably 3 - 40, especially 5 - 15 chosen from RNA complementary to the RNA corresponding to any of the genes and the use threof for in vitro analysis of allergy or tissue irritation.

Further it regards a reagent kit comprising one or more probes that recognize products produced during the expression of any the genes.

In various aspects and embodiments, the invention relates to the following:
1. A process for in vitro evaluation of a potentially allergenic or tissue irritating substance, characterised in that test cells are cultivated in the presence of the substance, and the presence of up regulated genes chosen from G1P2, OASL, IFIT1, TRIM22, IFI44L, MXI, RSAD2, IFIT3, IFITM1, IFIT2, SPR, GNB2, XK, IFITM3, C 33.28 HERV-H protein mRNA, IFITM3, XK, GPR15, MT1G, MT1B;MT1A, ADFP, IL8, MT1E, MT1F, MT1H, SLC30A1, SERPINB2, CD83, TncRNA or or expression products from them are measured.
2. The process according to paragraph 1, characterised in that the expression of one or more of G1P2, OASL, IFIT1, TRIM22, IFI44L, MXI, RSAD2, IFIT3, IFITM1, IFIT 2, indicates Type I allergy; one or more of SPR, GNB2, XK, IFITM3, indicates non allergy; one or more of C 33.28 HERV-H protein mRNA, IFITM3, XK, GPR15, indicates TYPE I/IV haptenes and one or more of MT1G, MT1B;MT1A, ADFP, IL8, MT1E, MT1F, XK, IFITM3, MT1H, SLC30A1, SERPINB2, GNB2, MTIB, CD83, TncRNA genes indicates Type IV allergy.
3. The process according to any of paragraphs 1-2, characterised in that RNA, DNA, amino acids, peptides or proteins are measured.
4. The process according to any of paragraphs 1-3, characterised in that the test cells are chosen from primary blood cells; whole blood, peripheral blood, lymphocytes, monocytes, and cells cultivated in vitro derived from blood cells or cell lines cultivated in vitro.
5. The process according to any of paragraphs 1-4, characterised in that the highest concentration of the substance being non toxic to the cells is serial diluted.
6. The process according to any of paragraphss 1 - 5, characterised in that cell proliferation is measured.
7. The process according to any of paragraphs 1-6, characterised in that genes correlated with interferon production are selected as an indication of class I immune response.
8. The process according to paragraph 7, characterised in that one or more of the genes G1P2, OASL, IFIT1, TRIM22, IFI44L, MXI, RSAD2, IFIT3, IFITM1 and IFIT2 are measured.
9. The process according to any of paragraphs 1-6, characterised in that the presence of genes up regulating IL-8 are measured, whereby the presence of high levels of genes up regulating IL-8 is an indication of an allergenic response.
10. The process according to any of paragraphs 1-6, characterised in that the presence of genes up regulated by neopterin are measured, whereby the presence of high levels of genes up regulated by neopterin is an indication of an allergenic response.
11. The process according to any of paragraphs 1-10, characterised in that; genes up regulated by Aspergillus are selected as indication of class I immune response.
12. Use of the expression products from one or more of the following genes G1P2, OASL, IFIT1, TRIM22, IFI44L, MXI, RSAD2, IFIT3, IFITM1, IFIT2, SPR, GNB2, XK, IFITM3, C 33.28 HERV-H protein mRNA, IFITM3, XK, GPR15, MT1G, MT1B;MT1A, ADFP, IL8, MT1E, MT1F, MT1H, SLC30A1, SERPINB2, CD83, TncRNA for in vitro analysis of allergy or tissue irritation.
13. Use of at least 5, preferably at least 10 such as 3-50, 5-40 or 10-30 amino acids chosen from RNA or DNA complementary to the RNA or DNA corresponding to any of the following genes G1P2, OASL, IFIT1, TRIM22, IFI44L, MXI, RSAD2, IFIT3, IFITM1, IFIT2, SPR, GNB2, XK, IFITM3, C 33.28 HERV-H protein. mRNA, IFITM3, XK, GPR 15, MT1G, MT1B;MT1A, ADFP, IL8, MT1E, MT1F, MT1H, SLC30A1, SERPINB2, CD83, TncRNA as a probe for in vitro analysis of allergy or tissue irritation.
14. A reagent kit comprising one or more probes, characterised in that the probes recognize products produced during the expression of any of G1P2, OASL, IFIT1, TRIM22, IFI44L, MXI, RSAD2, IFIT3, IFITM1, IFIT2, SPR, GNB2, XK, IFITM3, C 33.28 HERV-H protein mRNA, IFITM3, XK, GPR15, MT1G, MT1B;MT1A, ADFP, IL8, MT1E, MT1F, MT1H, SLC30A1, SERPINB2, CD83, TncRNA.
15. The reagent kit according to paragraph 14, further also comprising test cells.

The invention is further elucidated with the following figures:
Figure 1 The endocytic processing pathway
Figure 2. Type I hypersensitivity
Figure 3. Type II hypersensitivity
Figure 4. Type III hypersensitivity
Figure 5. Type IV hypersensitivity

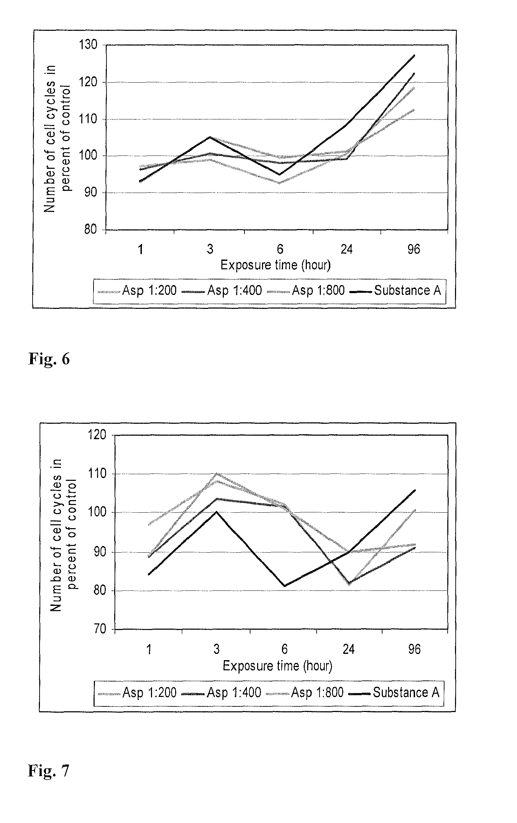

Figure 6. Number of cell cycles needed to get exponential expression of cGTP cyclohydrolas.
Figure 7. Number of cell cycles needed to get exponential expression of IL-8.

The following abbreviations are used in the description Abbreviations
- ADCC: antibody-dependent cell-mediated cytotoxicity
- APC: antigen presenting cell
- Asp: aspergillus fumigatus
- CPA: cytokine profile assay
- CPMP: committee for proprietary medicinal products
- DTH: delayed type hypersensitivity
- Fc: fold change
- GAPA: gene activation profile assay
- GPMT: guinea pig maximization test
- GTP: guanosine triphosphate
- ICCVAM: interagency coordinating committee on the validation of alternative methods
- IFN-γ: interferon gamma
- IL: interleukin
- LC: langerhans cell
- LLNA: local lymph node assay
- LPS: lipopolysaccaride
- MHC: major histocompability complex
- OECD: organization for economic cooperation and development
- PBMC: peripheral blood mononuclear cells
- RT-PCR: reverse transcription-polymerase chain reaction
- SDS: sodium dodecyl sulfonate
- TDI: toluene diisocyanate
- TNF: tumor necrosis factor

### Detailed description of the invention

The present invention relates to a process for in vitro evaluation of a potentially allergenic or tissue irritating substance whereby test cells are cultivated in the presence of the substance, and the presence of up regulated genes chosen from G1P2, OASL, IFIT1, TRIM22, IFI44L, MXI, RSAD2, IFIT3, IFITM1, IFIT2, SPR, GNB2, XK, IFITM3, C 33.28 HERV-H protein mRNA, IFITM3, XK, GPR15, MT1G, MT1B;MT1A, ADFP, IL8, MT1E, MT1F, MT1H, SLC30A1, SERPINB2, CD83, TncRNA or expression products from them are measured.

Especially the expression of one or more of G1P2, OASL, IFIT1, TRIM22, IFI44L, MXI, RSAD2, IFIT3, IFITM1, IFIT 2, indicates Type I allergy; one or more of SPR, GNB2, XK, IFITM3, indicates non allergy; one or more of C 33.28 HERV-H protein mRNA, IFITM3, XK, GPR15, indicates TYPE I/IV haptenes and one or more of MT1G, MT1B;MT1A, ADFP, IL8, MT1E, MT1F, XK, IFITM3, MT1H, SLC30A1, SERPINB2, GNB2, MTIB, CD83, TncRNA genes indicates Type IV allergy.

Expression product to be measured may be RNA, DNA, amino acids, peptides, proteins and derivatives thereof such as cDNA, or cRNA.

The gene sequences and the amino acid sequences for the corresponding genes of the above mentioned proteins are all known and can be found on GenBank (NIH genetic sequence data base)

According to one embodiment of the invention genes correlated with interferon production are selected as an indication of class I immune response. Such genes may be chosend form one or more of the genes G1P2, OASL, IFIT1, TRIM22, IFI44L, MXI, RSAD2, IFIT3, IFITM1 and IFIT2 are measured.

According to another embodiment of the invention the presence of genes up regulating IL-8 and neopterin respectively are measured, whereby the presence of high levels of genes up regulating IL-8 compared to genes up regulating neopterin, is an indication of class IV cell mediated T-cells immunity and delayed type hypersensitivity such as cellular immunity, delayed allergy and contact eczema.

It has turned out that genes that are up regulated by Aspergillus are indications of class I immune response.

According to another embodiment of the invention the presence of high levels of genes up regulating neopterin as well as genes up regulating IL-8, is an indication class I immune response type from T and B lymphocytes and inflammatory cells and immediate type hypersensitivity such as asthma, hay fever, urticaria and rhinitis.

The process according to the invention may be performed on test cells which may be chosen from primary blood cells; whole blood, peripheral blood, lymphocytes, monocytes, and cells cultivated in vitro derived from blood cells or cell lines cultivated in vitro. The highest concentration of the substance being non toxic to the cells may be serial diluted.

According to the invention cell proliferation may be established or inhibited and/or measured to get more expression products from the cells prior to measuring expressed genes. The proliferation may be done as described in WO 97/16732 and especially in the example thereof.

The invention also regards use of the expression products from one or more of the genes for in vitro analysis of allergy or tissue irritation.

For analysing expression product such as RNA, DNA and nucleic acids complementary to these sequences, cRNA and cDNA may be used as probes in a hybridisation test. At least 3 nucleic acids, such as at least 5, at least 10, at least 15 nucelic acids may be used as probes, such as 3-50, 5-40, 10-30 nucleic acids. The DNA sequences of the full genes may be found on GenBank. Useful probes are listed in materials and methods below. The invention also relates a reagent kit comprising on or more compartments comprising probes that recognize products produced during the expression of any of the above mentioned genes. There may also be compartments containing test cells or instruction notes.

While the invention has been described in relation to certain disclosed embodiments, the skilled person may foresee other embodiments, variations, or combinations which are not specifically mentioned but are nonetheless within the scope of the appended claims.

All references cited herein are hereby incorporated by reference in their entirety.

The expression "comprising" as used herein should be understood to include, but not be limited to, the stated items.

The invention will now be described by way of the following non-limiting examples.

### Examples

### Materials and methods

### Cell cultivation

The cell line Mono Mac 6 (AstraZeneca Cell Storage and Retrieval, Alderley Park, UK) was cultivated in RPMI 1640 medium with 10 mM HEPES buffer (Gibco, UK), 2 mM L-glutamine, 9 µg/mL human insulin, 1.0 mM sodium pyruvate, 10% fetal bovine serum, 5.6 µl/mL glucose, 100 U/mL penicillin and 100µg/mL streptomycin. Fresh medium for cultivation was added or changed frequently (every 2:nd or 3:rd day), maintaining a cell density of viable cells/mL between 0.5 x 10⁶ and 1.0 x 10⁶. The cell line was in suspension/loosely adherent and sub cultures were prepared when needed by scraping. The plates were cultivated in an inclined position at 37°C and 5% CO₂ in a Galaxy R (Lab Rum Klimat Ab, Sweden) incubator

### Viability counting

The remaining part of the cell suspension was used to calculate the viability. In experiment 041029 the cells were stained with Trypan Blue, and counted in a Bürker chamber using light microscopy. In experiment 041115 and 041213 a NucleoCounter™ (Chemometec, Denmark) was used.

### Test substances

### Time response study for the micro array analysis

Cell cultures were exposed to substances according to table 1, during 1, 3, 6, 24 and 96h. Control cell cultures were left unexposed.

**Table 1 Test substances in the kinetic experiment**

| **Substance** | **Concentration** | **Representing allergen class** |
|---|---|---|
| Aspergillus fumigatus | 1:200 | Allergen type I |
| Aspergillus fumigatus | 1:400 | Allergen type I |
| Aspergillus fumigatus | 1:800 | Allergen type I |
| Substance A¹⁾ | 50 µl/ml | Allergen type IV |

| | | |
|---|---|---|
| ¹⁾ Substance A, AstraZeneca, Sweden. Dissolved in distilled water. | | |

Preparation of total RNA was made according to RNeasy® Mini Handbook (Qiagen/VWR, Sweden). Real time polymerase chain reaction (PCR) was performed on a 7700 Sequence Detector System (Applied Biosystems, Sweden) using the Gene Expression Assay kit according to the manufactories (Applied Biosystems, USA). Probes and primers used was, the starting product for generating neopterin, GTP cyclohydrolase I (assay ID: Hs00609198_m, Applied Biosystems) and IL-8 (assay ID: Hs00174103_m1, Applied Biosystems). These genes served as positive control for allergic reactions. TaqMan analysis was performed according to standard operation procedures ("*Real Time PCR med TaqMan probe eller SYBR Green primers*", SAS 755-1, AstraZeneca, Sweden).

### Micro Array analysis

Cells were treated with four different allergens, according to table 2, in duplicate cultures. The test substances were all diluted in double distilled water. The duplicate cultures were treated at different exposure days. All treatments were 6 hours and control cells were left unexposed.

**Table 2 Substances used in experiment 041221 and 041222**

| **Substance** | **Concentration** | **Representing allergen class** |
|---|---|---|
| Penicillin G | 600 µg/ml | Allergen type I/IV, hapten |
| Substance A | 80 µg/ml | Allergen type IV, hapten |
| Albumin | 2 µg/ml | Non allergenic protein |
| Aspergillus | 1:200 | Allergen type I, protein |

Benzylpenicillin sodium salt (PenicillinG) was 13752, Sigma Aldrich, Germany; Albumin human was A9511, Sigma Aldrich, Germany and Aspergillus fumigatus was ALK15142 from Apoteket, Sweden and contained, except relevant allergen, also glycerol, sodium chloride, sodium hydrogen carbonate and water for injection ³³.

20 individual cultures with 500 000 cells per culture were treated identical for each substance. After 6h exposure identical treated cells were harvest and pooled into a 50 ml Falcontube, pelleted at 540g for 5 minutes in 7°C. The supernatant was discarded and the cells were washed in phosphate-buffered saline (with 0,5% bovine serum albumin), transferred to an eppendorftube and centrifuged at 150g for 2 minutes at room temperature. The supernatant was removed and the cells were freeze-dried with liquid nitrogen and thereafter put into -152°C freezer until further preparation.

Experimental procedures were performed according to *Gene Chip®Expression Analysis Technical Manual* (rev1, 2001) with minor modifications as described. Total RNA was prepared from frozen cells, according to Qiagen Rneasy Mini kit (Qiagen/WVR, Sweden). 30 µg of total RNA was used for cDNA synthesis and in vitro transcript labeling with biotin was performed according to Enzo BioArray RNA Transcription Labeling Kit (Enzo, U.S.A). cRNA quality was analyzed on a Agilent Bioanalyser 2001 (Agilent Technologies, U.S.A) and the concentration was measured on a Nano Droop (Saveen Werner, Sweden). 15 µg of fragmented cRNA was added to the hybridization-cocktail and hybridized to the HG_U95Av2 chip (Affymetrix, U.S.A) for 16h at 45°C. The arrays were washed and stained with biotinylated anti streptavidin antibodies according to the EukGE_W2v4 protocol (Affymetrix, U.S.A) in the fluid station (Affymetrix, U.S.A).

Data obtained were analyzed using the MAS 5.0 model base. A detection call was calculated for all probe sets, representing if the transcript of a particular gene was present or absent, all absent genes were excluded from analysis.
To verify outliers and trends in data exploratory analysis was made with principle component analysis (PCA). Statistic analysis was made using student's t-test. It weights the variance in individual groups with the variance in all groups. Student's t-test was used to test for statistical significance compared with control. The p-value obtained describes the probability of statistically finding a false positive probe set. Fold change (fc) represents the quotient between the two compared chip.

The average signal value from all treated groups were compared with control signals.

During the reading process of the chip an error occur for one of the chips representing material from penicillin G treated cells. Further analysis of this chip was inappropriate and the chip was excluded. Two of the chips, background and aspergillus, hade a very bad quality and were excluded. These chips were washed in the same washing station indicating that something was wrong with the equipment.

### Filter criteria

All probe sets with signal value <50 in all groups were excluded from analysis. Only probe sets that showed statistical significant up regulation (p<0.05) as compared to control, were included in the analysis. The remaining probe sets were ranked after fc.

### Cell cultivation

In the present study, a higher amount of glucose was needed to keep the cells growing. Otherwise, the cultivation conditions in the two studies were identical.

### Batches of allergen

Even though the allergens used in this study are standardized there might be a difference in composition between batches used for testing. These were different between the two studies.

### Stability of the cell line

The cell line used in the two studies was taken from the same supplier and also from the same passage. However, the studies were performed 1.5 years apart and the stability of the cell line might differ.

### HG-U95AV2 Affymetrix Probe Sequences

Information of the probe- sequences is reached at NETAFFX™ ANALYSIS CENTER (https://www.affymetrix.com/analysis/netaffx).

### Original Sequence Source: GenBank

Probes for the following genes are listed:

**Genes**

| | |
|---|---|
| 1 | GIP2 (ISG15) |
| 2 | OASL |
| 3 | IFIT1 |
| 4 | TRIM22 |
| 5 | IFI44L |
| 6 | MXI |
| 7 | RSAD2 |
| 8 | IFIT3 |
| 9 | IFITM1 |
| 10 | IFIT2 |
| 11 | SPR |
| 12 | GNB2 |
| 13 | XK |
| 14 | IFITM3 |
| 15 | GPR15 |
| 16 | MT1G |
| 17 | MT1B |
| 18 | MT1A |
| 19 | ADFP |
| 20 | IL-8 |
| 21 | MT1E |
| 22 | MT1F |
| 23 | MT1H |
| 24 | SLC30A1 |
| 25 | SERPINB2 |

### GIP2 (ISG15)

| **Probe Set: HG-U95AV2:1107_S_AT** | | | | |
|---|---|---|---|---|
| **Probe Sequences (5'- 3')** | **Probe X** | **Probe Y** | **Probe Interrogation** | **PositionTarget Strandedness** |
| AGAGGCAGCGAACTCATCTTTGCCA | 369 | 467 | 19 | Antisense |
| GGCGGGCAACGAATTCCAGGTGTCC | 465 | 511 | 117 | Antisense |
| TCCCTGAGCAGCTCCATGTCGGTGT | 478 | 471 | 139 | Antisense |
| TCCATGTCGGTGTCAGAGCTGAAGG | 562 | 331 | 151 | Antisense |
| AGCTGAAGGCGCAGATCACCCAGAA | 310 | 447 | 167 | Antisense |
| ACGCCTTCCAGCAGCGTCTGGCTGT | 590 | 375 | 203 | Antisense |
| GCGTCTGGCTGTCCACCCGAGCGGT | 313 | 599 | 216 | Antisense |
| GGACAAATGCGACGAACCTCTGAGC | 631 | 335 | 312 | Antisense |
| CGAACCTCTGAGCATCCTGGTGAGG | 354 | 397 | 324 | Antisense |
| GACCGTGGCCCACCTGAAGCAGCAA | 310 | 499 | 393 | Antisense |
| ACCTGAAGCAGCAAGTGAGCGGGCT | 506 | 575 | 404 | Antisense |
| GACGACCTGTTCTGGCTGACCTTCG | 615 | 511 | 442 | Antisense |
| CTGGCTGACCTTCGAGGGGAAGCCC | 484 | 423 | 453 | Antisense |
| AGTACGGCCTCAAGCCCCTGAGCAC | 470 | 515 | 503 | Antisense |
| TGAGCACCGTGTTCATGAATCTGCG | 230 | 631 | 521 | Antisense |
| CTCCACCAGCATCCGAGCAGGATCA | 314 | 577 | 590 | Antisense |

| **Probe Set: HG-U95Av2:38432_AT** | | | | |
|---|---|---|---|---|
| **Probe Sequences (5'- 3')** | **Probe X** | **Probe Y** | **Probe Interrogation** | **PositionTarget Strandedness** |
| TGACGCAGACCGTGGCCCACCTGAA | 180 | 457 | 452 | Antisense |
| GACCGTGGCCCACCTGAAGCAGCAA | 497 | 73 | 459 | Antisense |
| CTGGCTGACCTTCGAGGGGAAGCCC | 453 | 117 | 519 | Antisense |
| GGCTGACCTTCGAGGGGAAGCCCCT | 518 | 633 | 521 | Antisense |
| GAGTACGGCCTCAAGCCCCTGAGCA | 366 | 39 | 569 | Antisense |
| CAAGCCCCTGAGCACCGTGTTCATG | 62 | 445 | 580 | Antisense |
| GAGCACCGTGTTCATGAATCTGCGC | 483 | 167 | 589 | Antisense |
| CACCAGCATCCGAGCAGGATCAAGG | 13 | 489 | 660 | Antisense |
| AGCATCCGAGCAGGATCAAGGGCCG | 276 | 339 | 664 | Antisense |
| CGAGCAGGATCAAGGGCCGGAAATA | 607 | 221 | 670 | Antisense |
| TCAAGGGCCGGAAATAAAGGCTGTT | 472 | 631 | 679 | Antisense |
| GGTAATTTACTTGCATGCCGCTGTT | 494 | 223 | 761 | Antisense |
| CATGCCGCTGTTTAAATGTACTGGA | 166 | 329 | 774 | Antisense |
| AGAACCGTTCCGATGGTATAGAAGC | 510 | 593 | 820 | Antisense |
| CGTGCGTCTAAATCCATGATGCATG | 392 | 189 | 848 | Antisense |
| TTGGTTTCCCAAAAGGGTGCCTGAT | 549 | 555 | 936 | Antisense |

### OASL

| **Probe Set: HG-U95AV2:269_AT** | | | | |
|---|---|---|---|---|
| **Probe Sequences (5'- 3')** | **Probe X** | **Probe Y** | **Probe Interrogation** | **PositionTarget Strandedness** |
| ATGGACCTGCTCCTGGAGTATGAAG | 575 | 297 | 25 | Antisense |
| CTGCTCCTGGAGTATGAAGTCATCT | 493 | 303 | 31 | Antisense |
| TATGAAGTCATCTGTATCTACTGGA | 496 | 353 | 43 | Antisense |
| TACTACACACTCCACAATGCAATCA | 623 | 313 | 73 | Antisense |
| AGATGGGACATCGTTGCTCAGAGGG | 427 | 423 | 187 | Antisense |
| GACATCGTTGCTCAGAGGGCCTCCC | 586 | 413 | 193 | Antisense |
| CAGTGCCTGAAACAGGACTGTTGCT | 378 | 423 | 217 | Antisense |
| CTGAAACAGGACTGTTGCTATGACA | 503 | 511 | 223 | Antisense |
| TCCAGCTGGAACGTGAAGAGGGCAC | 281 | 511 | 265 | Antisense |
| AGGGCACGAGACATCCACTTGACAG | 405 | 583 | 283 | Antisense |
| ATCCACTTGACAGTGGAGCAGAGGG | 352 | 503 | 295 | Antisense |
| CCAGGGGCTACTCTGGCCTGCAGCG | 523 | 509 | 391 | Antisense |
| GCTACTCTGGCCTGCAGCGTCTGTC | 449 | 353 | 397 | Antisense |
| CTGGCCTGCAGCGTCTGTCCTTCCA | 391 | 505 | 403 | Antisense |
| TGCAGCGTCTGTCCTTCCAGGTTCC | 406 | 501 | 409 | Antisense |
| AGGTTCCTGGCAGTGAGAGGCAGCT | 376 | 557 | 427 | Antisense |

| **Probe Set: HG-U95Av2:34491_AT** | | | | |
|---|---|---|---|---|
| **Probe Sequences (5'- 3')** | **Probe X** | **Probe Y** | **Probe Interrogation** | **PositionTarget Strandedness** |
| CTTAGCCAAATATGGGATCTTCTCC | 158 | 145 | 1255 | Antisense |
| CCACACTCACATCTATCTGCTGGAG | 16 | 105 | 1279 | Antisense |
| ACATCTATCTGCTGGAGACCATCCC | 97 | 187 | 1287 | Antisense |
| CCCTCCGAGATCCAGGTCTTCGTGA | 299 | 225 | 1310 | Antisense |
| GATCCAGGTCTTCGTGAAGAATCCT | 72 | 263 | 1318 | Antisense |
| AGGTCTTCGTGAAGAATCCTGATGG | 259 | 543 | 1323 | Antisense |
| CTTCGTGAAGAATCCTGATGGTGGG | 288 | 617 | 1327 | Antisense |
| TTGGGTCTGGGGATCTATGGCATCC | 470 | 485 | 1480 | Antisense |
| GGGATCTATGGCATCCAAGACAGTG | 61 | 505 | 1489 | Antisense |
| GCATCCAAGACAGTGACACTCTCAT | 431 | 63 | 1499 | Antisense |
| AGACAGTGACACTCTCATCCTCTCG | 28 | 85 | 1506 | Antisense |
| TGACACTCTCATCCTCTCGAAGAAG | 96 | 107 | 1512 | Antisense |
| CCTCTCGAAGAAGAAAGGAGAGGCT | 73 | 255 | 1524 | Antisense |
| CTCTGGGAGACTTCTCTGTACATTT | 1 | 263 | 1571 | Antisense |
| GACTTCTCTGTACATTTCTGCCATG | 40 | 31 | 1579 | Antisense |
| GCCATGTACTCCAGAACTCATCCTG | 31 | 477 | 1598 | Antisense |

### IFIT1

| **Probe Set: HG-U95AV2:32814_AT** | | | | |
|---|---|---|---|---|
| **Probe Sequences (5'- 3')** | **Probe X** | **Probe Y** | **Probe Interrogation** | **PositionTarget Strandedness** |
| CATGAAACCAGTGGTAGAAGAAACA | 26 | 559 | 1194 | Antisense |
| TGCAAGACATACATTTCCACTATGG | 538 | 123 | 1220 | Antisense |
| CTATGGTCGGTTTCAGGAATTTCAA | 525 | 613 | 1239 | Antisense |
| AATAGAACAGGCATCATTAACAAGG | 29 | 483 | 1311 | Antisense |
| CAGGCATCATTAACAAGGGATAAAA | 196 | 333 | 1318 | Antisense |
| CATTAGATCTGGAAAGCTTGAGCCT | 107 | 537 | 1392 | Antisense |
| AAGCTTGAGCCTCCTTGGGTTCGTC | 49 | 437 | 1405 | Antisense |
| GCTTGAGCCTCCTTGGGTTCGTCTA | 520 | 633 | 1407 | Antisense |
| GCCTCCTTGGGTTCGTCTACAAATT | 168 | 383 | 1413 | Antisense |
| CCTCCTTGGGTTCGTCTACAAATTG | 169 | 383 | 1414 | Antisense |
| CGGGCCCTGAGACTGGCTGCTGACT | 166 | 405 | 1475 | Antisense |
| TGGCTGCTGACTTTGAGAACTCTGT | 149 | 521 | 1488 | Antisense |
| CTGCTGACTTTGAGAACTCTGTGAG | 305 | 251 | 1491 | Antisense |
| GACTTTGAGAACTCTGTGAGACAAG | 112 | 419 | 1496 | Antisense |
| TTGAGAACTCTGTGAGACAAGGTCC | 298 | 207 | 1500 | Antisense |
| ACTCTGTGAGACAAGGTCCTTAGGC | 511 | 33 | 1506 | Antisense |

| **Probe Set: HG-U95AV2:915_AT** | | | | |
|---|---|---|---|---|
| **Probe Sequences (5'- 3')** | **Probe X** | **Probe Y** | **Probe Interrogation** | **PositionTarget Strandedness** |
| TAAGATCAGCCATATTTCATTTTGA | 267 | 279 | 1041 | Antisense |
| AGCCCACATTTGAGGTGGCTCATCT | 386 | 253 | 1083 | Antisense |
| AGGTGGCTCATCTAGACCTGGCAAG | 277 | 439 | 1095 | Antisense |
| CTCATCTAGACCTGGCAAGAATGTA | 44 | 377 | 1101 | Antisense |
| CAATGCAAGACATACATTTCTACTA | 525 | 69 | 1203 | Antisense |
| AAGACATACATTTCTACTATGGTCG | 390 | 205 | 1209 | Antisense |
| ATCTGGAAAGCTTGAGCCTCCTTGG | 365 | 469 | 1383 | Antisense |
| ATATGAATGAAGCCCTGGAGTACTA | 320 | 339 | 1431 | Antisense |
| ATGAGCGGGCCCTGAGACTGGCTGC | 512 | 89 | 1455 | Antisense |
| TGGCTGCTGACTTTGAGAACTCTGT | 150 | 521 | 1473 | Antisense |
| TTGAGAACTCTGTGAGACAAGGTCC | 470 | 3 | 1485 | Antisense |
| ACTCTGTGAGACAAGGTCCTTAGGC | 510 | 33 | 1491 | Antisense |
| CTTAGGCACCCAGATATCAGCCACT | 594 | 487 | 1509 | Antisense |
| CACCCAGATATCAGCCACTTTCACA | 329 | 345 | 1515 | Antisense |
| GATATCAGCCACTTTCACATTTCAT | 304 | 297 | 1521 | Antisense |
| TTTATGCTAACATTTACTAATCATC | 634 | 357 | 1551 | Antisense |

### TRIM22

| **Probe Set: HG-U95AV2:36825_AT** | | | | |
|---|---|---|---|---|
| **Probe Sequences (5'- 3')** | **Probe X** | **Probe Y** | **Probe Interrogation** | **PositionTarget Strandedness** |
| CTTGGTTTCACTAGTAGTAAACATT | 228 | 231 | 2243 | Antisense |
| CCTCTGCCCCTTAAAAGATTGAAGA | 216 | 249 | 2362 | Antisense |
| CTCTGCCCCTTAAAAGATTGAAGAA | 431 | 107 | 2363 | Antisense |
| TGCCCCTTAAAAGATTGAAGAAAGA | 284 | 373 | 2366 | Antisense |
| GCCCCTTAAAAGATTGAAGAAAGAG | 283 | 373 | 2367 | Antisense |
| CACGTTATCTAGCAAAGTACATAAG | 227 | 233 | 2411 | Antisense |
| CCTTCAGAATGTGTTGGTTTACCAG | 349 | 539 | 2458 | Antisense |
| GAATGTGTTGGTTTACCAGTGACAC | 542 | 33 | 2464 | Antisense |
| ATGTGTTGGTTTACCAGTGACACCC | 403 | 25 | 2466 | Antisense |
| TGGTTTACCAGTGACACCCCATATT | 424 | 491 | 2472 | Antisense |
| GGTTTACCAGTGACACCCCATATTC | 405 | 303 | 2473 | Antisense |
| TTTAATGCTCAGAGTTTCTGAGGTC | 49 | 167 | 2551 | Antisense |
| AATGCTCAGAGTTTCTGAGGTCAAA | 321 | 213 | 2554 | Antisense |
| CTCAGAGTTTCTGAGGTCAAATTTT | 328 | 113 | 2558 | Antisense |
| AGCCATTTCAATGTCTTGGGAAACA | 145 | 161 | 2788 | Antisense |
| GCCATTTCAATGTCTTGGGAAACAA | 164 | 381 | 2789 | Antisense |

### IFI44L

| **Probe Set: HG-U95AV2:36927_AT** | | | | |
|---|---|---|---|---|
| **Probe Sequences (5'- 3')** | **Probe X** | **Probe Y** | **Probe Interrogation** | **PositionTarget Strandedness** |
| CAGCCCTGCATTTGAGATAAGTTGC | 128 | 607 | 1487 | Antisense |
| AAGTTGCCTTGATTCTGACATTTGG | 198 | 581 | 1505 | Antisense |
| CCTTGATTCTGACATTTGGCCCAGC | 330 | 493 | 1511 | Antisense |
| CCTGTACTGGTGTGCCGCAATGAGA | 195 | 553 | 1535 | Antisense |
| TTGACAGCCTGCTTCAGATTTTGCT | 321 | 449 | 1571 | Antisense |
| CAGCCTGCTTCAGATTTTGCTTTTG | 184 | 609 | 1575 | Antisense |
| TGCCTTCTGTCCTTGGAACAGTCAT | 452 | 269 | 1607 | Antisense |
| CTGTCCTTGGAACAGTCATATCTCA | 592 | 401 | 1613 | Antisense |
| AAGGCCAAAACCTGAGAAGCGGTGG | 499 | 507 | 1644 | Antisense |
| GGCTAAGATAGGTCCTACTGCAAAC | 310 | 557 | 1668 | Antisense |
| AGATAGGTCCTACTGCAAACCACCC | 593 | 397 | 1673 | Antisense |
| CTGTGACATCTTTTTAAACCACTGG | 365 | 375 | 1731 | Antisense |
| TGTGACATCTTTTTAAACCACTGGA | 403 | 291 | 1732 | Antisense |
| ATAACACTCTATATAGAGCTATGTG | 577 | 83 | 1790 | Antisense |
| CTCTATATAGAGCTATGTGAGTACT | 319 | 339 | 1796 | Antisense |
| GTATAGACATCTGCTTCTTAAACAG | 452 | 333 | 1852 | Antisense |

### MXI

| **Probe Set: HG-U95AV2:39072_AT** | | | | |
|---|---|---|---|---|
| **Probe Sequences (5'- 3')** | **Probe X** | **Probe Y** | **Probe Interrogation** | **PositionTarget Strandedness** |
| GTTAAGTTCAGCACTTGTCTCATTT | 424 | 539 | 2110 | Antisense |
| GTTCAGCACTTGTCTCATTTTAATG | 477 | 205 | 2115 | Antisense |
| GCACTTGTCTCATTTTAATGTAAAG | 555 | 33 | 2120 | Antisense |
| AGATTTGCTTCCATTTTCCTACAGG | 473 | 611 | 2143 | Antisense |
| TTTGCTTCCATTTTCCTACAGGCAG | 438 | 271 | 2146 | Antisense |
| GCTTCCATTTTCCTACAGGCAGTCT | 424 | 411 | 2149 | Antisense |
| AGGCAGTCTCTCTCTTCCTCACAGT | 614 | 187 | 2165 | Antisense |
| CTCACAGTCCCACTGTGCAGGTGCT | 474 | 139 | 2182 | Antisense |
| TCACAGTCCCACTGTGCAGGTGCTA | 438 | 131 | 2183 | Antisense |
| GTCCCACTGTGCAGGTGCTATTGTT | 92 | 509 | 2188 | Antisense |
| CTGTGCAGGTGCTATTGTTACTCTT | 260 | 567 | 2194 | Antisense |
| TGTGCAGGTGCTATTGTTACTCTTA | 241 | 457 | 2195 | Antisense |
| GTGCTATTGTTACTCTTACGAATAT | 540 | 183 | 2202 | Antisense |
| TCTTCTAAGTGAAATTTCTAGCCTG | 615 | 207 | 2244 | Antisense |
| TAAGTGAAATTTCTAGCCTGCACTT | 394 | 467 | 2249 | Antisense |
| CTGCACTTTGATGTCATGTGTTCCC | 529 | 171 | 2266 | Antisense |

| **Probe Set: HG-U95AV2:654_AT** | | | | |
|---|---|---|---|---|
| **Probe Sequences (5'- 3')** | **Probe X** | **Probe Y** | **Probe Interrogation** | **PositionTarget Strandedness** |
| ATCTATTTTGATGCAGCATTTGATA | 488 | 577 | 1917 | Antisense |
| ACCTCACTCTTTATAGTGCACAAAA | 455 | 471 | 1959 | Antisense |
| TTACCAGCTTTTAACCATCTGATAT | 354 | 451 | 2049 | Antisense |
| GCTTTTAACCATCTGATATCTATAG | 406 | 397 | 2055 | Antisense |
| GTAGACACACTATCATAGTTAACAT | 441 | 355 | 2079 | Antisense |
| ACACTATCATAGTTAACATAGTTAA | 599 | 289 | 2085 | Antisense |
| TAGTTAAGTTCAGCACTTGTCTCAT | 545 | 545 | 2103 | Antisense |
| AGTTCAGCACTTGTCTCATTTTAAT | 522 | 403 | 2109 | Antisense |
| TGTAAAGATTTGCTTCCATTTTCCT | 495 | 521 | 2133 | Antisense |
| CTTCCATTTTCCTACAGGCAGTCTC | 425 | 411 | 2145 | Antisense |
| CACTGTGCAGGTGCTATTGTTACTC | 453 | 341 | 2187 | Antisense |
| TTTCTAGCCTGCACTTTGATGTCAT | 398 | 471 | 2253 | Antisense |
| GCCTGCACTTTGATGTCATGTGTTC | 446 | 477 | 2259 | Antisense |
| ACTTTGATGTCATGTGTTCCCTTTG | 592 | 253 | 2265 | Antisense |
| TGTGTTCCCTTTGTCTTTCAAACTC | 293 | 565 | 2277 | Antisense |
| TCTTGGAGACCTTACCCCTGGCTGT | 382 | 591 | 2343 | Antisense |

| **Probe Set: HG-U95AV2:748_S_AT** | | | | |
|---|---|---|---|---|
| **Probe Sequences (5'- 3')** | **Probe X** | **Probe Y** | **Probe Interrogation** | **PositionTarget Strandedness** |
| AATCGACGAGCTCATCTGCGCCTTT | 599 | 209 | 136 | Antisense |
| TGCGCCTTTGTTTAGAACGCTTAAA | 527 | 569 | 152 | Antisense |
| GATTCCACTAGGACCAGACTGCACC | 500 | 537 | 183 | Antisense |
| CGGCACACAACACTTGGTTTGCTCA | 589 | 355 | 208 | Antisense |
| CCAGCTCGAGAATTTGGAACGAGAA | 470 | 573 | 288 | Antisense |
| TGGAACAGCTGCAGGGTCCTCAGGA | 321 | 549 | 335 | Antisense |
| ATACGAATGGACAGCATTGGATCAA | 464 | 553 | 370 | Antisense |
| CAGATCGTTCTGATTCAGAGCGAGA | 582 | 563 | 404 | Antisense |
| GAAAGCACAGAGTTCTCCCATGGAG | 276 | 561 | 448 | Antisense |
| ACCAGCATCAGTGACATTGATGACC | 607 | 325 | 493 | Antisense |
| TATTGGGAGTGACGAGGGTTACTCC | 599 | 345 | 534 | Antisense |
| CAGTGCCAGTGTCAAACTTTCATTC | 519 | 631 | 558 | Antisense |
| AGCATGACATAACAGTGCAGGGCAA | 474 | 311 | 597 | Antisense |
| TTCACTGGGCCAATTCAATACAAAC | 486 | 395 | 626 | Antisense |
| CAAACAATCTCTTAAATTGGGTTCA | 581 | 245 | 646 | Antisense |
| GGTTCATGATGCAGTCTCCTCTTTA | 371 | 465 | 665 | Antisense |

### RSAD2

| **Probe Set: HG-U95AV2:38549_AT** | | | | |
|---|---|---|---|---|
| **Probe Sequences (5'- 3')** | **Probe X** | **Probe Y** | **Probe Interrogation** | **PositionTarget Strandedness** |
| GTGGTACCTGTTGTGTCCCTTTCTC | 539 | 603 | 2604 | Antisense |
| TGTAGTTGAGTAGCTGGTTGGCCCT | 119 | 365 | 2759 | Antisense |
| GTTGAGTAGCTGGTTGGCCCTACAT | 74 | 417 | 2763 | Antisense |
| AGAGAGTGCCTGGATTTCATGTCAG | 9 | 59 | 2877 | Antisense |
| CCTGGATTTCATGTCAGTGAAGCCA | 16 | 69 | 2885 | Antisense |
| CTCTGAGTCAGTTGAAATAGGGTAC | 264 | 537 | 2937 | Antisense |
| TAGGGTACCATCTAGGTCAGTTTAA | 199 | 321 | 2954 | Antisense |
| ACCATCTAGGTCAGTTTAAGAAGAG | 221 | 125 | 2960 | Antisense |
| AGTCAGCTCAGAGAAAGCAAGCATA | 68 | 129 | 2983 | Antisense |
| GTCAGCTCAGAGAAAGCAAGCATAA | 98 | 115 | 2984 | Antisense |
| AAATGTCACGTAAACTAGATCAGGG | 60 | 83 | 3013 | Antisense |
| AATGTCACGTAAACTAGATCAGGGA | 49 | 535 | 3014 | Antisense |
| CTCTCCTTGTGGAAATATCCCATGC | 187 | 235 | 3047 | Antisense |
| TGGAAATATCCCATGCAGTTTGTTG | 136 | 227 | 3056 | Antisense |
| TATCCCATGCAGTTTGTTGATACAA | 43 | 25 | 3062 | Antisense |
| CCCATGCAGTTTGTTGATACAACTT | 49 | 67 | 3065 | Antisense |

### IFIT3

| **Probe Set: HG-U95AV2:38584_AT** | | | | |
|---|---|---|---|---|
| **Probe Sequences (5'- 3')** | **Probe X** | **Probe Y** | **Probe Interrogation** | **PositionTarget Strandedness** |
| TATTTTCCTGTCAGCATCTGAGCTT | 142 | 55 | 1472 | Antisense |
| CAGCATCTGAGCTTGAGGATGGTAG | 8 | 411 | 1483 | Antisense |
| GGCCAGGGCGCAGTCAGCTCCAGTC | 303 | 89 | 1518 | Antisense |
| CGCAGTCAGCTCCAGTCCCAGAGAG | 167 | 21 | 1526 | Antisense |
| AGTCAGCTCCAGTCCCAGAGAGCTC | 95 | 35 | 1529 | Antisense |
| CCAGAGAGCTCCTCTCTAACTCAGA | 107 | 39 | 1543 | Antisense |
| GCTCCTCTCTAACTCAGAGCAACTG | 59 | 89 | 1550 | Antisense |
| CTCTAACTCAGAGCAACTGAACTGA | 17 | 447 | 1556 | Antisense |
| CTCAGAGCAACTGAACTGAGACAGA | 240 | 1 | 1562 | Antisense |
| CTGAACTGAGACAGAGGAGGAAAAC | 201 | 565 | 1572 | Antisense |
| AACAGAGCATCAGAAGCCTGCAGTG | 47 | 109 | 1594 | Antisense |
| ATCAGAAGCCTGCAGTGGTGGTTGT | 109 | 351 | 1602 | Antisense |
| CCCAACCTGGGATTGCTGAGCAGGG | 260 | 75 | 1657 | Antisense |
| CAGGGAAGCTTTGCATGTTGCTCTA | 112 | 173 | 1677 | Antisense |
| AGCTTTGCATGTTGCTCTAAGGTAC | 28 | 75 | 1683 | Antisense |
| GCATGTTGCTCTAAGGTACATTTTT | 36 | 65 | 1689 | Antisense |

### IFITM1

| **Probe Set: HG-U95AV2:675_AT** | | | | |
|---|---|---|---|---|
| **Probe Sequences (5'- 3')** | **Probe X** | **Probe Y** | **Probe Interrogation** | **PositionTarget Strandedness** |
| TTCCCCAAAGCCAGAAGATGCACAA | 403 | 569 | 312 | Antisense |
| TCTTCTTGAACTGGTGCTGTCTGGG | 154 | 491 | 462 | Antisense |
| GATTCATCCTGTCACTGGTATTCGG | 168 | 635 | 624 | Antisense |
| TCCTGTCACTGGTATTCGGCTCTGT | 2 | 631 | 630 | Antisense |
| TATTCGGCTCTGTGACAGTCTACCA | 267 | 555 | 642 | Antisense |
| TGACAGTCTACCATATTATGTTACA | 340 | 629 | 654 | Antisense |
| TCTACCATATTATGTTACAGATAAT | 410 | 481 | 660 | Antisense |
| CCTGCAACCTTTGCACTCCACTGTG | 396 | 375 | 720 | Antisense |
| ACCTTTGCACTCCACTGTGCAATGC | 200 | 399 | 726 | Antisense |
| GCACTCCACTGTGCAATGCTGGCCC | 381 | 315 | 732 | Antisense |
| CTGGCCCTGCACGCTGGGGCTGTTG | 56 | 631 | 750 | Antisense |
| CTGCCCCTAGATACAGCAGTTTATA | 151 | 527 | 792 | Antisense |
| ACAGCAGTTTATACCCACACACCTG | 481 | 237 | 804 | Antisense |
| GTTTATACCCACACACCTGTCTACA | 605 | 135 | 810 | Antisense |
| ACCCACACACCTGTCTACAGTGTCA | 533 | 149 | 816 | Antisense |
| ACACCTGTCTACAGTGTCATTCAAT | 394 | 187 | 822 | Antisense |

| **Probe Set: HG-U95AV2:676_G_AT** | | | | |
|---|---|---|---|---|
| **Probe Sequences (5'- 3')** | **Probe X** | **Probe Y** | **Probe Interrogation** | **PositionTarget Strandedness** |
| GACCATGTCGTCTGGTCCCTGTTCA | 463 | 283 | 431 | Antisense |
| CATGTCGTCTGGTCCCTGTTCAACA | 618 | 349 | 434 | Antisense |
| TCGTCTGGTCCCTGTTCAACACCCT | 509 | 89 | 438 | Antisense |
| TCTGGTCCCTGTTCAACACCCTCTT | 416 | 381 | 441 | Antisense |
| GGGCTTCATAGCATTCGCCTACTCC | 64 | 615 | 484 | Antisense |
| GCTTCATAGCATTCGCCTACTCCGT | 509 | 121 | 486 | Antisense |
| ATAGCATTCGCCTACTCCGTGAAGT | 550 | 127 | 491 | Antisense |
| GCATTCGCCTACTCCGTGAAGTCTA | 409 | 573 | 494 | Antisense |
| GCCTACTCCGTGAAGTCTAGGGACA | 478 | 287 | 500 | Antisense |
| TACTCCGTGAAGTCTAGGGACAGGA | 494 | 503 | 503 | Antisense |
| CTCCGTGAAGTCTAGGGACAGGAAG | 230 | 433 | 505 | Antisense |
| GCGACGTGACCGGGGCCCAGGCCTA | 422 | 451 | 537 | Antisense |
| CACCGCCAAGTGCCTGAACATCTGG | 187 | 603 | 568 | Antisense |
| CGCCAAGTGCCTGAACATCTGGGCC | 549 | 403 | 571 | Antisense |
| CCAAGTGCCTGAACATCTGGGCCCT | 520 | 221 | 573 | Antisense |
| AGTGCCTGAACATCTGGGCCCTGAT | 357 | 525 | 576 | Antisense |

### IFIT2

| **Probe Set: HG-U95AV2:908_AT** | | | | |
|---|---|---|---|---|
| **Probe Sequences (5'- 3')** | **Probe X** | **Probe Y** | **Probe Interrogation** | **PositionTarget Strandedness** |
| AAATTGCCAAAATGCGACTTTCTAA | 467 | 609 | 1262 | Antisense |
| CCAAAATGCGACTTTCTAAAAATGG | 463 | 449 | 1268 | Antisense |
| AAATGCGACTTTCTAAAAATGGAGC | 564 | 449 | 1271 | Antisense |
| TGCGACTTTCTAAAAATGGAGCAGA | 427 | 387 | 1274 | Antisense |
| GAGCAGATTCTGAGGCTTTGCATGT | 412 | 423 | 1292 | Antisense |
| ATTCTGAGGCTTTGCATGTCTTGGC | 277 | 509 | 1298 | Antisense |
| CTGAGGCTTTGCATGTCTTGGCATT | 482 | 609 | 1301 | Antisense |
| AGGCTTTGCATGTCTTGGCATTCCT | 580 | 555 | 1304 | Antisense |
| CTTTGCATGTCTTGGCATTCCTTCA | 445 | 399 | 1307 | Antisense |
| ATGTCTTGGCATTCCTTCAGGAGCT | 513 | 587 | 1313 | Antisense |
| TCTTGGCATTCCTTCAGGAGCTGAA | 262 | 541 | 1316 | Antisense |
| CATTCCTTCAGGAGCTGAATGAAAA | 451 | 433 | 1322 | Antisense |
| AAATGCAACAAGCAGATGAAGACTC | 236 | 559 | 1346 | Antisense |
| GTTTGGAGTCTGGAAGCCTCATCCC | 308 | 467 | 1379 | Antisense |
| AGTCTGGAAGCCTCATCCCTTCAGC | 350 | 555 | 1385 | Antisense |
| CTGGAAGCCTCATCCCTTCAGCATC | 389 | 451 | 1388 | Antisense |

| **Probe Set: HG-U95AV2:909_G_AT** | | | | |
|---|---|---|---|---|
| **Probe Sequences (5'- 3')** | **Probe X** | **Probe Y** | **Probe Interrogation** | **PositionTarget Strandedness** |
| CAAAGCGATTGAACTGCTTAAAAAG | 541 | 247 | 804 | Antisense |
| TTGCCAAATTGGGTGCTGCTATAGG | 541 | 579 | 864 | Antisense |
| GCAAAAGTCTTCCAAGTAATGAATC | 317 | 635 | 889 | Antisense |
| AACTAATAGGACACGCTGTGGCTCA | 524 | 341 | 953 | Antisense |
| AAGCTGATGAGGCCAATGATAATCT | 461 | 463 | 986 | Antisense |
| TCCGTGTCTGTTCCATTCTTGCCAG | 517 | 303 | 1013 | Antisense |
| GCCTCCATGCTCTAGCAGATCAGTA | 474 | 563 | 1037 | Antisense |
| TCTAGCAGATCAGTATGAAGACGCA | 558 | 301 | 1047 | Antisense |
| TACTTCCAAAAGGAATTCAGTAAAG | 382 | 429 | 1078 | Antisense |
| AGCTTACTCCTGTAGCGAAACAACT | 622 | 445 | 1103 | Antisense |
| TGTAGCGAAACAACTGCTCCATCTG | 450 | 563 | 1113 | Antisense |
| AACTGCTCCATCTGCGGTATGGCAA | 517 | 411 | 1124 | Antisense |
| ATCTGCGGTATGGCAACTTTCAGCT | 458 | 425 | 1133 | Antisense |
| GGCAACTTTCAGCTGTACCAAATGA | 578 | 467 | 1144 | Antisense |
| CAGCTGTACCAAATGAAGTGTGAAG | 563 | 217 | 1153 | Antisense |
| GACAAGGCCATCCACCACTTTATAG | 580 | 491 | 1177 | Antisense |

### SPR

| **Probe Set: HG-U95AV2:32108_AT** | | | | |
|---|---|---|---|---|
| **Probe Sequences (5'- 3')** | **Probe X** | **Probe Y** | **Probe Interrogation** | **PositionTarget Strandedness** |
| AGCCCATGTTTTTGGCTTCCTGAAC | 432 | 397 | 824 | Antisense |
| CATGTTTTTGGCTTCCTGAACCTTT | 304 | 143 | 828 | Antisense |
| ACACCCTGCCATAGGGGCAGTCCTG | 39 | 327 | 896 | Antisense |
| TAGAAGCATTCATGCCTGCTGCCCT | 66 | 325 | 930 | Antisense |
| TGCCCTCAGGCACAGCCAGCTGTGA | 102 | 147 | 954 | Antisense |
| CACCCTGGGTTATAAGGAGGCTTAG | 30 | 309 | 1025 | Antisense |
| TTATGGGTATTGGTGTCTCTATCCC | 322 | 225 | 1058 | Antisense |
| GTCTCTATCCCCAGGAATAGAACTT | 222 | 95 | 1072 | Antisense |
| TATCCCCAGGAATAGAACTTAAGGG | 267 | 361 | 1077 | Antisense |
| AGAGGAGGTTGTGTCTCTTGCTCAT | 230 | 143 | 1138 | Antisense |
| CATAGCAAGCCTGTGGGTAGAGGAA | 398 | 51 | 1160 | Antisense |
| TGATCTGGTGTCGAATAGGAGGACC | 53 | 105 | 1189 | Antisense |
| TCTGGTGTCGAATAGGAGGACCCAT | 615 | 15 | 1192 | Antisense |
| ATAGGAGGACCCATGTAGATTCGCA | 180 | 155 | 1203 | Antisense |
| TGTAGATTCGCAGATGGCCTGGATG | 96 | 181 | 1216 | Antisense |
| AGCCCACATAGATGCCCCTTGCTGA | 40 | 107 | 1268 | Antisense |

### GNB2

| **Probe Set: HG-U95AV2:38831_F_AT** | | | | |
|---|---|---|---|---|
| **Probe Sequences (5'- 3')** | **Probe X** | **Probe Y** | **Probe Interrogation** | **PositionTarget Strandedness** |
| GGCTACGACGACTTCAACTGCAACA | 126 | 315 | 1133 | Antisense |
| GCTACGACGACTTCAACTGCAACAT | 511 | 21 | 1134 | Antisense |
| CCTTCCTCAAGATCTGGAACTAATG | 315 | 223 | 1287 | Antisense |
| CTTCCTCAAGATCTGGAACTAATGG | 429 | 15 | 1288 | Antisense |
| TTCCTCAAGATCTGGAACTAATGGC | 417 | 145 | 1289 | Antisense |
| TCCTCAAGATCTGGAACTAATGGCC | 407 | 111 | 1290 | Antisense |
| CCTCAAGATCTGGAACTAATGGCCC | 408 | 111 | 1291 | Antisense |
| CTCAAGATCTGGAACTAATGGCCCC | 498 | 541 | 1292 | Antisense |
| GCAGGAGGCCCTCATCCTTCTGCTG | 142 | 295 | 1528 | Antisense |
| TCATCCTTCTGCTGCCCTGGGGTTG | 37 | 507 | 1539 | Antisense |
| CAGTTTTTCCATAAAGGAGCCAATT | 612 | 369 | 1659 | Antisense |
| CATAAAGGAGCCAATTCCAACTCTG | 459 | 133 | 1668 | Antisense |
| | | | | |
| | | | | |
| | | | | |
| | | | | |

| **Probe Set: HG-U95AV2:38832_R_AT** | | | | |
|---|---|---|---|---|
| **Probe Sequences (5'- 3')** | **Probe X** | **Probe Y** | **Probe Interrogation** | **PositionTarget Strandedness** |
| TCCCGGGGCCCCCACTGTGGAGATA | 564 | 225 | 1473 | Antisense |
| GGGGCCCCCACTGTGGAGATAAGAA | 280 | 621 | 1477 | Antisense |
| CCCCCACTGTGGAGATAAGAAGGGG | 427 | 15 | 1481 | Antisense |
| AGGAGCAGGAGGCCCTCATCCTTCT | 377 | 237 | 1524 | Antisense |
| GAGCAGGAGGCCCTCATCCTTCTGC | 175 | 355 | 1526 | Antisense |
| CAGGAGGCCCTCATCCTTCTGCTGC | 141 | 295 | 1529 | Antisense |
| AGGCCCTCATCCTTCTGCTGCCCTG | 252 | 221 | 1533 | Antisense |
| CCTCATCCTTCTGCTGCCCTGGGGT | 317 | 323 | 1537 | Antisense |
| CTTCTGCTGCCCTGGGGTTGGGGCC | 369 | 171 | 1544 | Antisense |
| TCTGCTGCCCTGGGGTTGGGGCCTC | 173 | 411 | 1546 | Antisense |
| TGCTGCCCTGGGGTTGGGGCCTCAC | 252 | 579 | 1548 | Antisense |
| GCTGCCCTGGGGTTGGGGCCTCACC | 253 | 579 | 1549 | Antisense |
| TTTATTATATTTTCAGTTTTTCCAT | 53 | 431 | 1646 | Antisense |
| TATTATATTTTCAGTTTTTCCATAA | 48 | 431 | 1648 | Antisense |
| TTATATTTTCAGTTTTTCCATAAAG | 128 | 581 | 1650 | Antisense |
| TATTTTCAGTTTTTCCATAAAGGAG | 149 | 469 | 1653 | Antisense |

### XK

| **Probe Set: HG-U95AV2:40647_AT** | | | | |
|---|---|---|---|---|
| **Probe Sequences (5'- 3')** | **Probe X** | **Probe Y** | **Probe Interrogation** | **PositionTarget Strandedness** |
| TCTTTGGTCTTCTCGACAGGTGCCC | 310 | 175 | 4757 | Antisense |
| GTCTTCTCGACAGGTGCCCTTTCTC | 88 | 371 | 4763 | Antisense |
| CCACTGAATCTGAGAAAGTACTTTC | 377 | 129 | 4847 | Antisense |
| TGGAAACCACCTTAAAACATTAGTG | 537 | 305 | 5056 | Antisense |
| CACCTTAAAACATTAGTGCTATGGT | 138 | 479 | 5063 | Antisense |
| ACCTTAAAACATTAGTGCTATGGTT | 139 | 479 | 5064 | Antisense |
| GTGTATGTGCCAGTACTTACCAGTC | 550 | 149 | 5093 | Antisense |
| ATGTGCCAGTACTTACCAGTCAATG | 428 | 121 | 5097 | Antisense |
| TGCCAGTACTTACCAGTCAATGCAT | 272 | 491 | 5100 | Antisense |
| ACCAGTCAATGCATTGTGGATATGA | 421 | 51 | 5111 | Antisense |
| GGATATGAGCTTTCGTTGACTGCTT | 355 | 155 | 5128 | Antisense |
| TATGAGCTTTCGTTGACTGCTTCTC | 408 | 21 | 5131 | Antisense |
| AGCTTTCGTTGACTGCTTCTCTGCA | 2 | 383 | 5135 | Antisense |
| TTCGTTGACTGCTTCTCTGCAGTCG | 281 | 189 | 5139 | Antisense |
| TTGACTGCTTCTCTGCAGTCGTTGA | 111 | 303 | 5143 | Antisense |
| CTCTGCAGTCGTTGATGCTAATAAA | 80 | 407 | 5153 | Antisense |

### IFITM3

| **Probe Set: HG-U95AV2:41745_AT** | | | | |
|---|---|---|---|---|
| **Probe Sequences (5'- 3')** | **Probe X** | **Probe Y** | **Probe Interrogation** | **PositionTarget Strandedness** |
| CTTCTCTCCTGTCAACAGTGGCCAG | 476 | 135 | 274 | Antisense |
| CCGACCATGTCGTCTGGTCCCTGTT | 353 | 601 | 420 | Antisense |
| GACCATGTCGTCTGGTCCCTGTTCA | 464 | 283 | 422 | Antisense |
| CCATGTCGTCTGGTCCCTGTTCAAC | 387 | 409 | 424 | Antisense |
| CATGTCGTCTGGTCCCTGTTCAACA | 619 | 349 | 425 | Antisense |
| CGGAGCCGAGTCCTGTATCAGCCCT | 51 | 591 | 788 | Antisense |
| GAGCCGAGTCCTGTATCAGCCCTTT | 481 | 477 | 790 | Antisense |
| GCCGAGTCCTGTATCAGCCCTTTAT | 281 | 601 | 792 | Antisense |
| CCGAGTCCTGTATCAGCCCTTTATC | 282 | 601 | 793 | Antisense |
| GAGTCCTGTATCAGCCCTTTATCCT | 131 | 515 | 795 | Antisense |
| TTCTACAATGGCATTCAATAAAGTG | 265 | 363 | 829 | Antisense |
| CTACAATGGCATTCAATAAAGTGCA | 572 | 79 | 831 | Antisense |
| TACAATGGCATTCAATAAAGTGCAC | 357 | 253 | 832 | Antisense |
| CAATGGCATTCAATAAAGTGCACGT | 243 | 435 | 834 | Antisense |
| ATTCAATAAAGTGCACGTGTTTCTG | 594 | 285 | 841 | Antisense |
| TCAATAAAGTGCACGTGTTTCTGGT | 499 | 573 | 843 | Antisense |

### GPR15

| **Probe Set: HG-U95AV2:31426_AT** | | | | |
|---|---|---|---|---|
| **Probe Sequences (5'- 3')** | **Probe X** | **Probe Y** | **Probe Interrogation** | **PositionTarget Strandedness** |
| GTTGCCTACTCTTCTGTCCAGGGAG | 335 | 347 | 507 | Antisense |
| ATACTGTGCAGAGAAAAAGGCAACT | 41 | 357 | 555 | Antisense |
| GGCAACTCCAATTAAACTCATATGG | 188 | 185 | 573 | Antisense |
| TCCCTGGTGGCCTTAATTTTCACCT | 277 | 449 | 598 | Antisense |
| TTTGTCCCTTTGTTGAGCATTGTGA | 360 | 31 | 625 | Antisense |
| TACCAGCAATCAGGAAAGCACAACA | 32 | 119 | 688 | Antisense |
| TAAAGATCATCTTTATTGTCGTGGC | 158 | 259 | 731 | Antisense |
| TTTCTTGTCTCCTGGCTGCCCTTCA | 212 | 173 | 760 | Antisense |
| GGCTGCCCTTCAATACTTTCAAGTT | 91 | 149 | 773 | Antisense |
| GTTCCTGGCCATTGTCTCTGGGTTG | 466 | 213 | 795 | Antisense |
| GTGAGTGGACCCTTGGCATTTGCCA | 240 | 17 | 868 | Antisense |
| GGCATTTGCCAACAGCTGTGTCAAC | 246 | 389 | 882 | Antisense |
| ATATCTTCGACAGCTACATCCGCCG | 345 | 199 | 920 | Antisense |
| ATCTTCGACAGCTACATCCGCCGGG | 207 | 81 | 922 | Antisense |
| CGCCGGGCCATTGTCCACTGCTTGT | 160 | 281 | 940 | Antisense |
| GACTTTGGGAGTAGCACTGAGACAT | 60 | 239 | 985 | Antisense |

### MT1G

| **Probe Set: HG-U95AV2:926_AT** | | | | |
|---|---|---|---|---|
| **Probe Sequences (5'- 3')** | **Probe X** | **Probe Y** | **Probe Interrogation** | **PositionTarget Strandedness** |
| TTCCCTTCTCGCTTGGGAACTCTAG | 566 | 443 | 43 | Antisense |
| TTCTCGCTTGGGAACTCTAGTCTCG | 305 | 603 | 48 | Antisense |
| TCGCTTGGGAACTCTAGTCTCGCCT | 217 | 429 | 51 | Antisense |
| CGCTTGGGAACTCTAGTCTCGCCTC | 218 | 429 | 52 | Antisense |
| GCTTGGGAACTCTAGTCTCGCCTCG | 570 | 559 | 53 | Antisense |
| TTGGGAACTCTAGTCTCGCCTCGGG | 144 | 453 | 55 | Antisense |
| TGGGAACTCTAGTCTCGCCTCGGGT | 340 | 235 | 56 | Antisense |
| GGGAACTCTAGTCTCGCCTCGGGTT | 630 | 605 | 57 | Antisense |
| AGCCCTGCTCCCAAGTACAAATAGA | 380 | 515 | 280 | Antisense |
| CCTGCTCCCAAGTACAAATAGAGTG | 221 | 457 | 283 | Antisense |
| TGCTCCCAAGTACAAATAGAGTGAC | 528 | 141 | 285 | Antisense |
| CTCCCAAGTACAAATAGAGTGACCC | 434 | 203 | 287 | Antisense |
| TCCCAAGTACAAATAGAGTGACCCG | 330 | 323 | 288 | Antisense |
| ATAGAGTGACCCGTAAAATCTAGGA | 541 | 357 | 300 | Antisense |
| TAGAGTGACCCGTAAAATCTAGGAT | 407 | 617 | 301 | Antisense |
| GTTTTTTGCTACAATCTTGACCCCT | 503 | 479 | 331 | Antisense |

### MT1B

| **Probe Set: HG-U95AV2:609_F_AT** | | | | |
|---|---|---|---|---|
| **Probe Sequences (5'- 3')** | **Probe X** | **Probe Y** | **Probe Interrogation** | **PositionTarget Strandedness** |
| ACTGCTCCTGCACCACAGGTGGCTC | 290 | 523 | 23 | Antisense |
| CTGCACCACAGGTGGCTCCTGTGCC | 300 | 497 | 30 | Antisense |
| CACAGGTGGCTCCTGTGCCTGCGCC | 597 | 215 | 36 | Antisense |
| CTGCGCCGGCTCCTGCAAGTGCAAA | 387 | 615 | 54 | Antisense |
| GGCTCCTGCAAGTGCAAAGAGTGCA | 424 | 605 | 61 | Antisense |
| AGTGCAAATGTACCTCCTGCAAGAA | 598 | 463 | 80 | Antisense |
| AAATGTACCTCCTGCAAGAAGTGCT | 461 | 617 | 85 | Antisense |
| TACCTCCTGCAAGAAGTGCTGCTGC | 590 | 457 | 90 | Antisense |
| CTGCAAGAAGTGCTGCTGCTCTTGC | 605 | 583 | 96 | Antisense |
| GCTGCTGCTCTTGCTGCCCCGTGGG | 365 | 479 | 107 | Antisense |
| TGCTGCCCCGTGGGCTGTGCCAAGT | 380 | 539 | 118 | Antisense |
| CCCCGTGGGCTGTGCCAAGTGTGCC | 171 | 623 | 123 | Antisense |
| GCTGTGCCAAGTGTGCCCAGGGCTG | 372 | 495 | 131 | Antisense |
| TGTGCCCAGGGCTGTGTCTGCAAAG | 608 | 267 | 142 | Antisense |
| CCAGGGCTGTGTCTGCAAAGGCTCA | 561 | 501 | 147 | Antisense |
| GCTGTGTCTGCAAAGGCTCATCAGA | 400 | 419 | 152 | Antisense |

### MT1A

| **Probe Set: HG-U95AV2:31623_F_AT** | | | | |
|---|---|---|---|---|
| **Probe Sequences (5'- 3')** | **Probe X** | **Probe Y** | **Probe Interrogation** | **PositionTarget Strandedness** |
| ACTCCTGCAAGAAGAGCTGCTGCTC | 169 | 49 | 92 | Antisense |
| CTCCTGCAAGAAGAGCTGCTGCTCC | 204 | 221 | 93 | Antisense |
| TCCTGCAAGAAGAGCTGCTGCTCCT | 3 | 239 | 94 | Antisense |
| CCTGCAAGAAGAGCTGCTGCTCCTG | 4 | 239 | 95 | Antisense |
| GCAAGAAGAGCTGCTGCTCCTGCTG | 265 | 55 | 98 | Antisense |
| CAAGAAGAGCTGCTGCTCCTGCTGC | 264 | 55 | 99 | Antisense |
| AGAAGAGCTGCTGCTCCTGCTGCCC | 262 | 53 | 101 | Antisense |
| CTGCTGCCCCATGAGCTGTGCCAAG | 349 | 23 | 117 | Antisense |
| TGCCCCATGAGCTGTGCCAAGTGTG | 225 | 77 | 121 | Antisense |
| CCCCATGAGCTGTGCCAAGTGTGCC | 224 | 77 | 123 | Antisense |
| ATGAGCTGTGCCAAGTGTGCCCAGG | 247 | 65 | 127 | Antisense |
| CTGTGCCAAGTGTGCCCAGGGCTGC | 5 | 467 | 132 | Antisense |
| CCAAGTGTGCCCAGGGCTGCATATG | 112 | 147 | 137 | Antisense |
| TGTGCCCAGGGCTGCATATGCAAAG | 277 | 133 | 142 | Antisense |
| TGCCCAGGGCTGCATATGCAAAGGG | 254 | 259 | 144 | Antisense |
| CCCAGGGCTGCATATGCAAAGGGGC | 253 | 259 | 146 | Antisense |

### ADFP

| **Probe Set: HG-U95AV2:34378_AT** | | | | |
|---|---|---|---|---|
| **Probe Sequences (5'- 3')** | **Probe X** | **Probe Y** | **Probe Interrogation** | **PositionTarget Strandedness** |
| ATCCTCAGCTGACTGAGTCTCAGAA | 190 | 477 | 1335 | Antisense |
| CTGAGTCTCAGAATGCTCAGGACCA | 268 | 487 | 1347 | Antisense |
| CTCAGAATGCTCAGGACCAAGGTGC | 219 | 571 | 1353 | Antisense |
| ATGCTCAGGACCAAGGTGCAGAGAT | 634 | 129 | 1359 | Antisense |
| GCCAGGAGACCCAGCGATCTGAGCA | 610 | 39 | 1395 | Antisense |
| CCTATCACTAGTGCATGCTGTGGCC | 567 | 193 | 1440 | Antisense |
| GCTGTGGCCAGACAGATGACACCTT | 144 | 585 | 1456 | Antisense |
| CAGATGACACCTTTTGTTATGTTGA | 324 | 329 | 1468 | Antisense |
| TGAAATTAACTTGCTAGGCAACCCT | 542 | 295 | 1490 | Antisense |
| ACTTGCTAGGCAACCCTAAATTGGG | 607 | 305 | 1498 | Antisense |
| GCTAGGCAACCCTAAATTGGGAAGC | 408 | 433 | 1502 | Antisense |
| TGTCTGCTCTGGTGTGATCTGAAAA | 184 | 475 | 1775 | Antisense |
| CTCTGGTGTGATCTGAAAAGGCGTC | 443 | 249 | 1781 | Antisense |
| CTGAAAAGGCGTCTTCACTGCTTTA | 179 | 585 | 1793 | Antisense |
| AGGCGTCTTCACTGCTTTATCTCAT | 594 | 343 | 1799 | Antisense |
| CACTGCTTTATCTCATGATGCTTGC | 232 | 471 | 1808 | Antisense |

### IL-8

| **Probe Set: HG-U95AV2:1369_S_AT** | | | | |
|---|---|---|---|---|
| **Probe Sequences (5' - 3')** | **Probe X** | **Probe Y** | **Probe Interrogation** | **PositionTarget Strandedness** |
| TTTTCCTAGATATTGCACGGGAGAA | 256 | 535 | 674 | Antisense |
| TATCCGAACTTTAATTTCAGGAATT | 427 | 505 | 736 | Antisense |
| AATGGGTTTGCTAGAATGTGATATT | 618 | 465 | 762 | Antisense |
| TTTTGCCATAAAGTCAAATTTAGCT | 469 | 495 | 820 | Antisense |
| TTTTCTGTTAAATCTGGCAACCCTA | 592 | 553 | 860 | Antisense |
| TTAAATCTGGCAACCCTAGTCTGCT | 564 | 505 | 867 | Antisense |
| CTGGCAACCCTAGTCTGCTAGCCAG | 386 | 547 | 873 | Antisense |
| CCCTAGTCTGCTAGCCAGGATCCAC | 635 | 621 | 880 | Antisense |
| GCTAGCCAGGATCCACAAGTCCTTG | 515 | 623 | 889 | Antisense |
| AGGATCCACAAGTCCTTGTTCCACT | 604 | 557 | 896 | Antisense |
| CACAAGTCCTTGTTCCACTGTGCCT | 317 | 547 | 902 | Antisense |
| CCTTGTTCCACTGTGCCTTGGTTTC | 630 | 205 | 909 | Antisense |
| AAAGTATTAGCCACCATCTTACCTC | 552 | 529 | 954 | Antisense |
| AGCCACCATCTTACCTCACAGTGAT | 609 | 453 | 962 | Antisense |
| ACATGTGGAAGCACTTTAAGTTTTT | 347 | 565 | 996 | Antisense |
| TTTAAGTTTTTTCATCATAACATAA | 350 | 627 | 1010 | Antisense |

| **Probe Set: HG-U95AV2:35372_R_AT** | | | | |
|---|---|---|---|---|
| **Probe Sequences (5'- 3')** | **Probe X** | **Probe Y** | **Probe Interrogation** | **PositionTarget Strandedness** |
| TATTTGTGCAAGAATTTGGAAAAAT | 528 | 79 | 1098 | Antisense |
| TAAATTTCAATCAGGGTTTTTAGAT | 446 | 621 | 1207 | Antisense |
| CCCAGTTAAATTTTCATTTCAGATA | 254 | 515 | 1254 | Antisense |
| AGTACATTATTGTTTATCTGAAATT | 637 | 315 | 1303 | Antisense |
| TAATTGAACTAACAATCCTAGTTTG | 369 | 617 | 1329 | Antisense |
| TGAACTAACAATCCTAGTTTGATAC | 351 | 319 | 1333 | Antisense |
| ACTAACAATCCTAGTTTGATACTCC | 110 | 591 | 1336 | Antisense |
| ACAATCCTAGTTTGATACTCCCAGT | 569 | 587 | 1340 | Antisense |
| ATCCTAGTTTGATACTCCCAGTCTT | 433 | 511 | 1343 | Antisense |
| TGGTAGTGCTGTGTTGAATTACGGA | 549 | 635 | 1385 | Antisense |
| TATTAAAACAGCCAAAACTCCACAG | 22 | 601 | 1425 | Antisense |
| CAGCCAAAACTCCACAGTCAATATT | 95 | 613 | 1433 | Antisense |
| CCAAAACTCCACAGTCAATATTAGT | 485 | 633 | 1436 | Antisense |
| ATATTAGTAATTTCTTGCTGGTTGA | 230 | 573 | 1453 | Antisense |
| TTAGTAATTTCTTGCTGGTTGAAAC | 444 | 503 | 1456 | Antisense |
| GTAATTTCTTGCTGGTTGAAACTTG | 557 | 487 | 1459 | Antisense |

### MT1E

| **Probe Set: HG-U95AV2:36130_F_AT** | | | | |
|---|---|---|---|---|
| **Probe Sequences (5'- 3')** | **Probe X** | **Probe Y** | **Probe Interrogation** | **PositionTarget Strandedness** |
| GCATCCCCTTTGCTCGAAATGGACC | 328 | 405 | 131 | Antisense |
| TGCTCGAAATGGACCCCAACTGCTC | 376 | 455 | 141 | Antisense |
| GAAATGGACCCCAACTGCTCTTGCG | 361 | 265 | 146 | Antisense |
| AAATGGACCCCAACTGCTCTTGCGC | 360 | 265 | 147 | Antisense |
| TGCTCTTGCGCCACTGGTGGCTCCT | 163 | 515 | 161 | Antisense |
| GCCACTGGTGGCTCCTGCACGTGCG | 496 | 279 | 170 | Antisense |
| ACTGGTGGCTCCTGCACGTGCGCCG | 564 | 365 | 173 | Antisense |
| ACGTGCGCCGGCTCCTGCAAGTGCA | 589 | 495 | 188 | Antisense |
| TGCGCCGGCTCCTGCAAGTGCAAAG | 390 | 217 | 191 | Antisense |
| TCCTGCAAGTGCAAAGAGTGCAAAT | 4 | 613 | 200 | Antisense |
| CATCGGAGAAGTGCAGCTGCTGTGC | 294 | 493 | 319 | Antisense |
| GAAGTGCAGCTGCTGTGCCTGATGT | 416 | 337 | 326 | Antisense |
| AAGTGCAGCTGCTGTGCCTGATGTG | 415 | 337 | 327 | Antisense |
| AGCTGCTGTGCCTGATGTGGGAACA | 330 | 427 | 333 | Antisense |
| CTGTGCCTGATGTGGGAACAGCTCT | 297 | 383 | 338 | Antisense |
| ATGTGGGAACAGCTCTTCTCCCAGA | 617 | 351 | 347 | Antisense |

### MT1F

| **Probe Set: HG-U95AV2:31622_F_AT** | | | | |
|---|---|---|---|---|
| **Probe Sequences (5' - 3')** | **Probe X** | **Probe Y** | **Probe Interrogation** | **PositionTarget Strandedness** |
| GTGTCTCCTGCACCTGCGCTGGTTC | 290 | 75 | 41 | Antisense |
| TGCACCTGCGCTGGTTCCTGCAAGT | 136 | 245 | 49 | Antisense |
| TCCTGCAAGTGCAAAGAGTGCAAAT | 236 | 103 | 64 | Antisense |
| AGAGTGCAAATGCACCTCCTGCAAG | 302 | 111 | 78 | Antisense |
| GCAAATGCACCTCCTGCAAGAAGAG | 182 | 279 | 83 | Antisense |
| AAATGCACCTCCTGCAAGAAGAGCT | 194 | 115 | 85 | Antisense |
| CTCCTGCAAGAAGAGCTGCTGCTCC | 203 | 221 | 93 | Antisense |
| TCCTGCAAGAAGAGCTGCTGCTCCT | 2 | 239 | 94 | Antisense |
| CCTGCAAGAAGAGCTGCTGCTCCTG | 1 | 241 | 95 | Antisense |
| AGAAGAGCTGCTGCTCCTGCTGCCC | 261 | 53 | 101 | Antisense |
| CCTGCTGCCCCGTGGGCTGTAGCAA | 396 | 151 | 116 | Antisense |
| CCCCGTGGGCTGTAGCAAGTGTGCC | 319 | 353 | 123 | Antisense |
| CCCGTGGGCTGTAGCAAGTGTGCCC | 34 | 451 | 124 | Antisense |
| CCGTGGGCTGTAGCAAGTGTGCCCA | 546 | 349 | 125 | Antisense |
| CTGTAGCAAGTGTGCCCAGGGCTGT | 4 | 467 | 132 | Antisense |
| TGTGCCCAGGGCTGTGTTTGCAAAG | 222 | 341 | 142 | Antisense |

### MT1H

| **Probe Set: HG-U95AV2:39594_F_AT** | | | | |
|---|---|---|---|---|
| **Probe Sequences (5'- 3')** | **Probe X** | **Probe Y** | **Probe Interrogation** | **PositionTarget Strandedness** |
| GGAACTCCAGTCTCACCTCGGCTTG | 221 | 207 | 43 | Antisense |
| TCCAGTCTCACCTCGGCTTGCAATG | 284 | 349 | 48 | Antisense |
| CTCGGCTTGCAATGGACCCCAACTG | 311 | 531 | 59 | Antisense |
| TCGGCTTGCAATGGACCCCAACTGC | 225 | 295 | 60 | Antisense |
| CTCCTGCGAGGCTGGTGGCTCCTGC | 46 | 87 | 84 | Antisense |
| GGCTCCTGCAAGTGCAAAAAGTGCA | 218 | 33 | 118 | Antisense |
| TCCTGCAAGTGCAAAAAGTGCAAAT | 135 | 285 | 121 | Antisense |
| AAAGTGCAAATGCACCTCCTGCAAG | 251 | 55 | 135 | Antisense |
| GCAAATGCACCTCCTGCAAGAAGAG | 18 | 7 | 140 | Antisense |
| AAATGCACCTCCTGCAAGAAGAGCT | 193 | 115 | 142 | Antisense |
| CTCCTGCAAGAAGAGCTGCTGCTCC | 80 | 51 | 150 | Antisense |
| TCCTGCAAGAAGAGCTGCTGCTCCT | 1 | 239 | 151 | Antisense |
| GAAGAGCTGCTGCTCCTGTTGCCCC | 31 | 277 | 159 | Antisense |
| TGCCCCCTGGGCTGTGCCAAGTGTG | 10 | 603 | 178 | Antisense |
| GTGCCCAGGGCTGCATCTGCAAAGG | 276 | 133 | 200 | Antisense |
| CCCAGGGCTGCATCTGCAAAGGGGC | 25 | 117 | 203 | Antisense |

### SLC30A1

| **Probe Set: HG-U95AV2:34759_AT** | | | | |
|---|---|---|---|---|
| **Probe Sequences (5' - 3')** | **Probe X** | **Probe Y** | **Probe Interrogation** | **PositionTarget Strandedness** |
| CAAATTGCCATGTTATGGTTCTGCC | 217 | 345 | 1877 | Antisense |
| GCCATGTTATGGTTCTGCCTTGAAA | 253 | 285 | 1883 | Antisense |
| TATGGTTCTGCCTTGAAACAGCACA | 268 | 221 | 1890 | Antisense |
| CTTGAAACAGCACAATGAAGTGTAT | 463 | 103 | 1901 | Antisense |
| TGAAACAGCACAATGAAGTGTATCA | 142 | 435 | 1903 | Antisense |
| TCTTCTGTTGCCTGTCCTTTGGGCC | 465 | 107 | 1972 | Antisense |
| TTGCCTGTCCTTTGGGCCAGATGTG | 510 | 167 | 1979 | Antisense |
| TTCATGACTGTGTGTTATTTTCCAA | 567 | 281 | 2095 | Antisense |
| TGACTGTGTGTTATTTTCCAAAGCT | 72 | 479 | 2099 | Antisense |
| TGTGTTATTTTCCAAAGCTGTTCCT | 244 | 337 | 2105 | Antisense |
| GTGTTATTTTCCAAAGCTGTTCCTA | 245 | 337 | 2106 | Antisense |
| AAAGCTGTTCCTACCTCACCATGAG | 179 | 389 | 2118 | Antisense |
| AGCTGTTCCTACCTCACCATGAGGC | 541 | 189 | 2120 | Antisense |
| GTTCCTACCTCACCATGAGGCTTTA | 217 | 611 | 2124 | Antisense |
| TACCTCACCATGAGGCTTTATGGAT | 498 | 39 | 2129 | Antisense |
| TCACCATGAGGCTTTATGGATTGTT | 436 | 237 | 2133 | Antisense |

### SERPINB2

| **Probe Set: HG-U95AV2:37185_AT** | | | | |
|---|---|---|---|---|
| **Probe Sequences (5'- 3')** | **Probe X** | **Probe Y** | **Probe Interrogation** | **PositionTarget Strandedness** |
| CTCACCCTAAAACTAAGCGTGCTGC | 106 | 119 | 1324 | Antisense |
| AAACTAAGCGTGCTGCTTCTGCAAA | 105 | 321 | 1333 | Antisense |
| AGCGTGCTGCTTCTGCAAAAGATTT | 581 | 29 | 1339 | Antisense |
| CTGCTTCTGCAAAAGATTTTTGTAG | 7 | 477 | 1345 | Antisense |
| TTTTTGTAGATGAGCTGTGTGCCTC | 268 | 93 | 1361 | Antisense |
| TTTGTAGATGAGCTGTGTGCCTCAG | 80 | 331 | 1363 | Antisense |
| GTGTGCCTCAGAATTGCTATTTCAA | 141 | 243 | 1377 | Antisense |
| GCCTCAGAATTGCTATTTCAAATTG | 77 | 399 | 1381 | Antisense |
| TCATTTGGTCTTCTAAAATGGGATC | 316 | 571 | 1526 | Antisense |
| TTGGTCTTCTAAAATGGGATCATGC | 460 | 471 | 1530 | Antisense |
| GGGATCATGCCCATTTAGATTTTCC | 263 | 189 | 1545 | Antisense |
| GGATCATGCCCATTTAGATTTTCCT | 18 | 237 | 1546 | Antisense |
| TTGCTCACTGCCTATTTAATGTAGC | 267 | 29 | 1648 | Antisense |
| GCTCACTGCCTATTTAATGTAGCTA | 354 | 23 | 1650 | Antisense |
| GCCTTTAATTGTTCTCATAATGAAG | 443 | 105 | 1722 | Antisense |
| AGTAGGTATCCCTCCATGCCCTTCT | 603 | 361 | 1751 | Antisense |

### SERPINB2

| **Probe Set: HG-U95AV2:37536_AT** | | | | |
|---|---|---|---|---|
| **Probe Sequences (5'- 3')** | **Probe X** | **Probe Y** | **Probe Interrogation** | **PositionTarget Strandedness** |
| GGGTGCTATCCATTTCTCATGTTTT | 149 | 71 | 1781 | Antisense |
| GGTGCTATCCATTTCTCATGTTTTC | 228 | 37 | 1782 | Antisense |
| TACCAAGAAGCCTTTCCTGTAGCCT | 630 | 505 | 1829 | Antisense |
| GAAGCCTTTCCTGTAGCCTTCTGTA | 472 | 25 | 1835 | Antisense |
| GCCTTCTGTAGGAATTCTTTTGGGG | 175 | 175 | 1850 | Antisense |
| TGAGGAAGCCAGGTCCACGGTCTGT | 203 | 203 | 1878 | Antisense |
| CACTCCAAGATATGGACACACGGGA | 133 | 55 | 1924 | Antisense |
| CTGGCAGAAGGGACTTCACGAAGTG | 467 | 137 | 1953 | Antisense |
| CTTCACGAAGTGTTGCATGGATGTT | 390 | 85 | 1966 | Antisense |
| GATGTTTTAGCCATTGTTGGCTTTC | 420 | 321 | 1985 | Antisense |
| GCCATTGTTGGCTTTCCCTTATCAA | 208 | 97 | 1994 | Antisense |
| TGGCTTTCCCTTATCAAACTTGGGC | 436 | 15 | 2002 | Antisense |
| TTCCCTTCTTGGTTTCCAAAGGCAT | 335 | 405 | 2029 | Antisense |
| TCCAAAGGCATTTTATTGCTTGAGT | 204 | 341 | 2043 | Antisense |
| TTGAGTTATATGTTCACTGTCCCCC | 190 | 391 | 2062 | Antisense |
| CTGTCTTGGCTTTCATGTTATTAAA | 110 | 67 | 2136 | Antisense |

### Example 1. Gene expression profiling of MonoMac 6 cells following allergen treatment.

To elucidate how fast an activation of the cells stimulated with an allergen occurs, a time response study of mRNA levels in the cells was made. The optimal exposure time was decided and cells were exposed to three different allergens and one non allergenic protein after which gene expression analysis was made.

### Results

### Gene expression profiling of MonoMac 6 cells following allergen treatment

The time response experiment was made to evaluate how fast the allergen affects the cells and an expression of allergen-related genes occur.

The number of cell cycles needed to get exponential expression of cGTP cyclohydrolas and IL-8 is shown in Figure 22 and 23, respectively. The fewer cell cycles needed to get an exponential expression of the gene the more RNA is present in the cell. An exposure time of 1 hour seams to be to short for the cell system to be stabilized and while neopterin (here represented by cGTP cyclohydrolas) has been shown to be a more interesting biomarker than IL-8, 6 hours was chosen to be the optimal exposure time.

Table 3 shows the number of regulated probe sets at different values of the fold change (fc) for each substance, following the filtrations described in materials and methods.

**Table 3. Number of up regulated genes at different cut of values for fc.**

| fc | Aspergillus | Albumin | Substance A | Penicillin G |
|---|---|---|---|---|
| > 2 | 94 | 16 | 16 | 4 |
| > 4 | 30 | 1 | 2 | 1 |
| > 6 | 24 | 0 | 0 | 0 |
| > 10 | 14 | 0 | 0 | 0 |

It is clear that cells exposed to aspergillus show a greater number of regulated genes than cells exposed to the other substances. The up regulation is also much stronger in aspergillus treated cultures compared to the other.

The 14 probe sets that were up regulated more than 10 times in aspergillus where evaluated and their gene products function were examined. These 14 probe sets code fore ten different genes. These and the probe set up regulated more than 2 times in albumin, substance A and penicillin G were examined. The genes correlated to the probe set, known biological process the gene products are participating in and their molecular function can be seen for aspergillus, albumin, substance A and penicillin G treated cells in table 4, 5, 6 and 7 respectively.

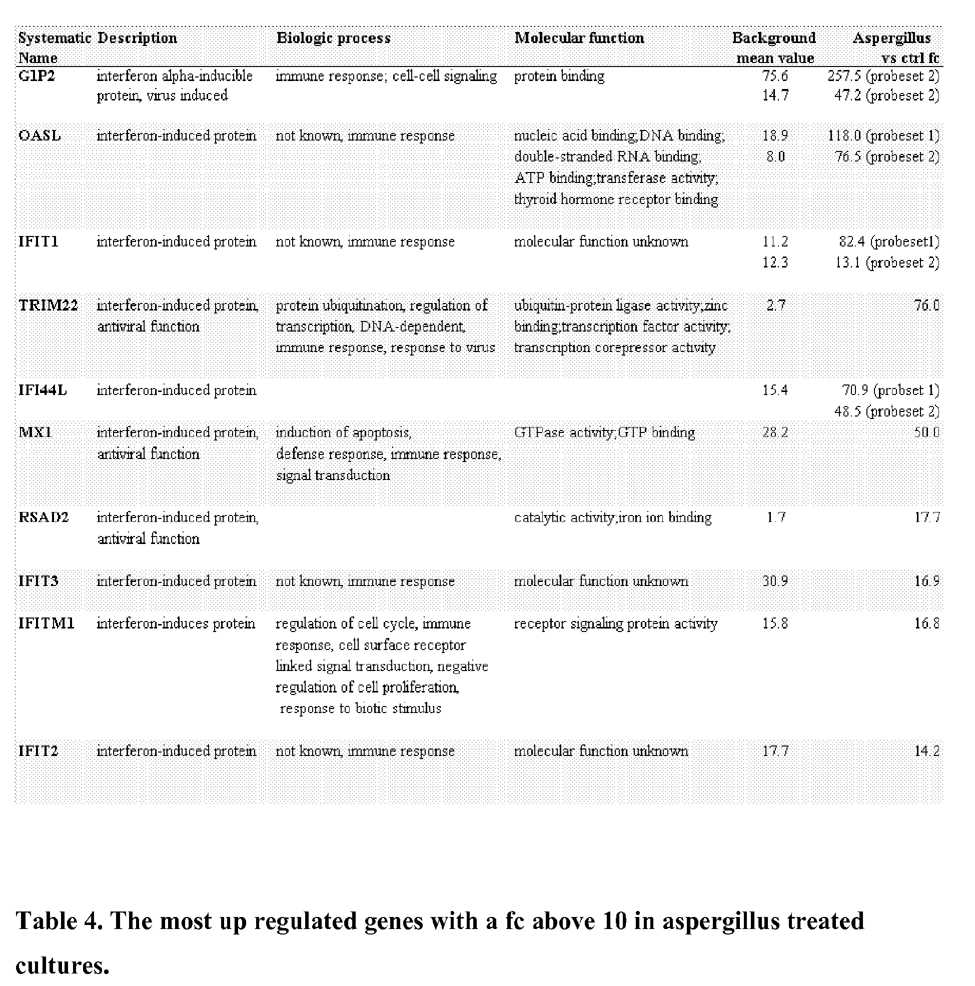

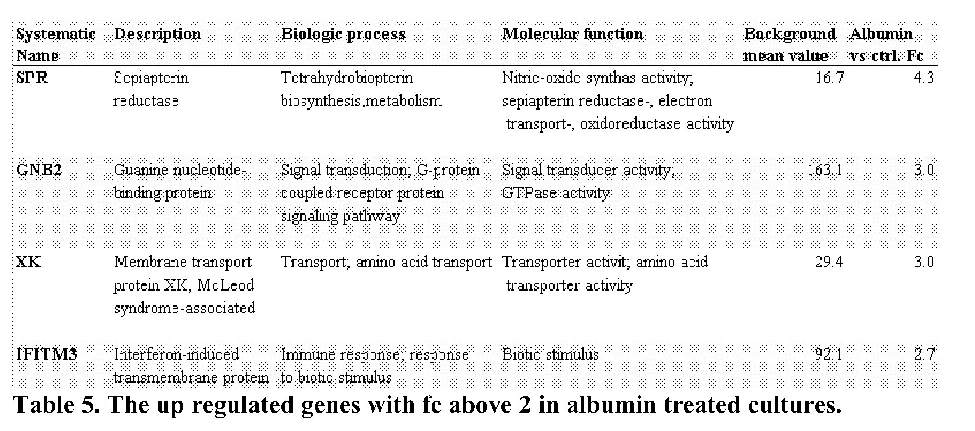

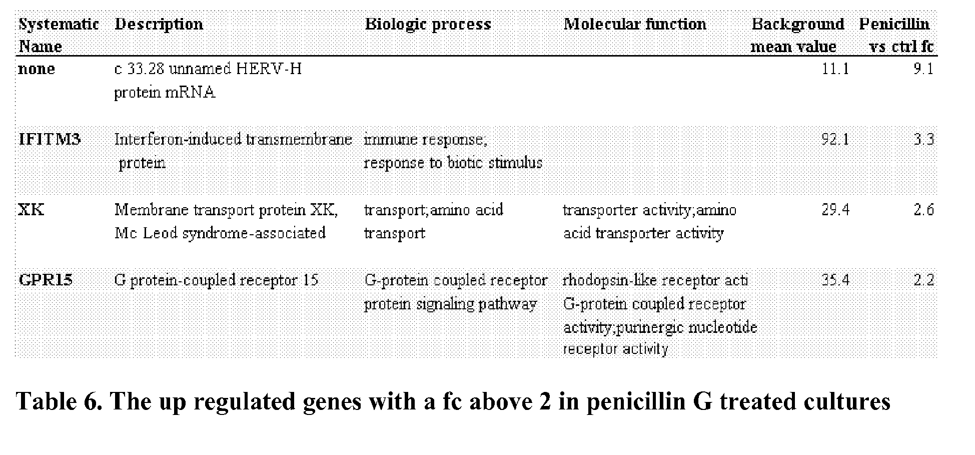

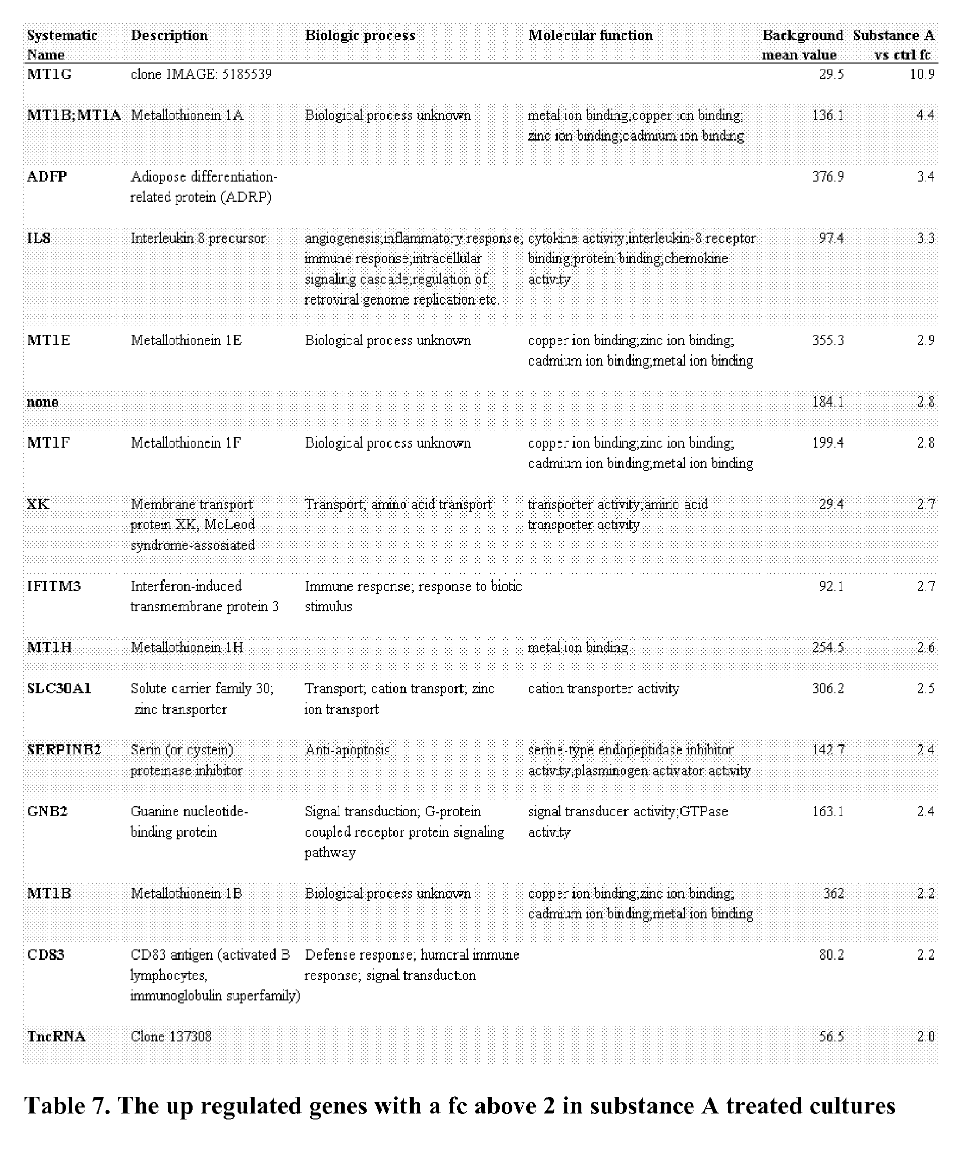

Notable is that all of the 10 genes that are most up regulated in aspergillus treated cultures are genes that have been shown to be interferon induced ^{23,24;25;26;27}.

The regulation of interferon's can be seen in Table 7, where most of them are down regulated.

Also notable is that five of the 16 genes up regulated more than two times in cell cultures treated with substance A are metallothioneins^{28;29}.

None of the 10 gene products up regulated in aspergillus treated cultures more than 10 times are up regulated more than 2 times in cell cultures treated with either albumin, substance A or penicillin G. IFITM3 and XK are both up regulated more than 2 times in cell cultures treated with substance A, penicillin G and albumin but not in aspergillus.

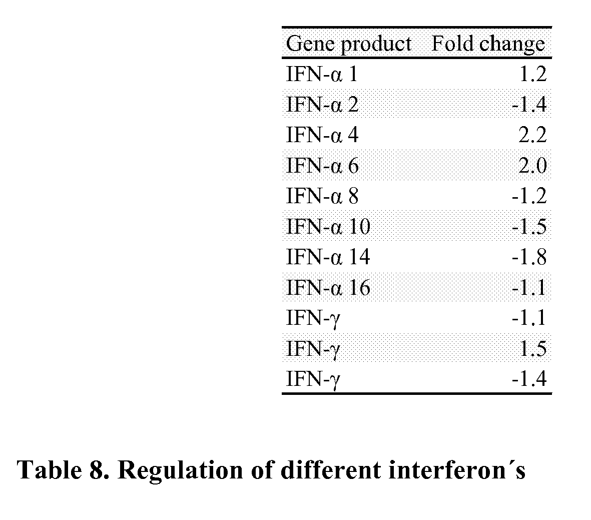

### Gene expression

There was a considerably greater up regulation of specific genes in cell cultures exposed to aspergillus compared to cultures treated with albumin, penicillin G and substance A. None of the 10 most up regulated genes, fc between 14 and 257, found in aspergillus treated cultures had a fc >2 in the other cultures.

All the up regulated genes in cell cultures treated with aspergillus were classified as interferon induced. The question is how this response could have been induced? Have a production of interferon occurred or is the interferon induced genes up regulated without an interferon production? It also has to be questioned if this happens general for all allergens or if it is specific for aspergillus.

Monocytes have been shown to secrete high levels of IFN-α, and, to a lesser degree, other forms of type-I IFN. IFN-α has a number of fundamental roles in innate and adaptive responses to pathogens. An increased secretion of IFN-α,β during the early phase of viral infection is well known but can also occur due to several other stimuli, such as bacteria and cytokines³⁰.

One possible scenario could be induction of interferon production due to similarities between aspergillus and viral capsid structures. If so, this would cause cells adjacent to the aspergillus presenting monocyte to initiate interferon production as in the case of a virus infection. Another possible mechanism could be that sequences of aspergillus, degraded and secreted from the cell, may have IFN-like structures able to bind IFN-receptors on the cells and induce IFN-regulated gene products. This may also be true for the non-degraded aspergillus protein. It could be questioned if all these reactions and responses are able to occur during six hours, as was the exposure time.

While aspergillus is a fungus the preparation of the fungal extract, that is not well characterized, could include some viral components. The activation of interferon can then be a response due to a viral affect in the aspergillus preparation³¹.

There are several examples of where the frequency of drug hypersensitivity is increased in the presence of a viral infection, for example is hypersensitivity reactions often observed by clinicians treating patients infected by human immunodeficiency virus (HIV)^{31;32}. This correlation can be an indication of that allergenic compound and virus infections have some pathways in common, and may be interesting to further elucidate. Supporting this theory is that four of the ten genes induced by exposure to aspergillus have an antiviral function.

Contradict this discussion is that MxA, a gene highly expressed in the aspergillus treated cultures is a reliable index of the production of type-I IFNs³³. However, MxA is not dependent on any external stimuli such as viral infection, thus a production of interferon has probably occurred. Another factor that speak for the "production of interferon" theory is that some interferon genes are up regulated even if the majority of the genes are down regulated in cell cultures exposed to aspergillus. However, the up regulated interferon producing genes are capable of inducing the interferon induced genes.

The first step in the production of neopterin is activation of cyclohydrolase I that is induced by interferon, mostly IFN-γ but also high concentrations of IFN-α or IFN-β. If neopterin is a useful biomarker for allergenic proteins then other substances correlated with the interferon production may be biomarkers also correlated to the allergenic protein.

Is this activation of interferon inducible genes only a response to the aspergillus protein or could it be a common mechanism for all or most allergenic proteins? Further studies are needed to confirm such a relationship.

Five of the 16 up regulated genes, fc >2, in cell cultures exposed to substance A coded for several kinds of metallothioneins. In man, metallothioneins comprise a multigene family consisting of about 10-12 members containing about 30% cysteins amino acids²⁸. Metallothioneins has been known for as long as about half a century, their precise physiological function is still under debate. Previously it has been shown that metallothioneins bind toxic metals, inhibiting the attack of free radicals and oxidative stress. The synthesis of these genes is induced by the metal ions to which they bind, i.e., Cd++, Zn++, Hg++, Cu++, Ag+ and Au+ or by treatment with glucocorticoids²⁹. More recently, Maret and Callee³⁴concluded that the role of metallothioneins lies in the control of the cellular zinc distribution as a function of the energy state of the cell. Substance A does not contain any metal ions, thus the induction of these genes cannot be due to metal ions. The answer of why substande A induce up regulation of metallothioneins needs to be further elucidated, is it a universal mechanism for type IV allergens or an effect due to merely substance A.

Some of the backgrounds values for the genes up regulated in aspergillus treated cultures are very low. Up regulations from values to low to be truly estimated are unreliable and a100-fold up regulation may with Real Time PCR appear to be a 4 time up regulation.

With a comparison between two groups with Students t-test there will be probe sets with a p-value below the 5% level just by chance. Decreasing the level of significance accepted can reduce the numbers of false positive answers. Some of the false positive answers are excluded when a criteria of the fc is set while the fc and the p-value is closely correlated. There will still be false positive probe sets in the remaining list and therefore the results have to be confirmed by more specific methods, for example Real Time PCR.

### Conclusion

The general up regulation of genes was more pronounced in cultures exposed to an allergenic proteins than to a non allergenic protein or to haptens.

All of the most up regulated genes in cultures exposed to allergenic protein were classified as interferon induced.

Many of the most up regulated genes in cells exposed to allergenic (type IV) hapten coded for metallothioneins.

### References

**1.** Johansson L and Andersson B. Development of a Predictive In Vitro Test for identification of Allergens: Evaluation of 15 Well Documented Allergenic or Skin Irritating Compounds. Biovator - Biological Innovations Inc. Sweden. (1995).
**2.** Goldsby R.A, K.T.J.O.B.A. Kuby Immunology. W.H.Freeman and company, New York (2000).
**3.** Astwood J.D, L.J.N.a.F.R.L. Stability of food allergens to digestion in vitro. Nature Biotechnology 14, 1269-73 (1996).
**4.** Marshall R.D. Glycoproteins. Annual Reviw of Biochemistry 41, 673-702 (1972).
**5.** Merget R, S.J.W.R.F.H.K.U.e.al. Diagnostic tests in enzyme allergy. Journal of Allergy & Clinical Immunology 92, 264-277 (1993).
**6.** Huby R.D.J, D.R.J.K.I. Why are some proteins allergens? Toxicological sciences 55, 235-246 (2000).
**7.** Lepoittevin J-P, B.D.G.A.K.A.-T. Allergic Contact Dermatitis. The Molecular Basis. Springer-Verlag, Berlin, Heidelberg, New York (1998).
**8.** Sinigaglia F. The Molecular Basis of Metal Recognition by T Cells. Journal of Investigative Dermatology 102, 398-401 (1994).
**9.** S.G.O. Johansson, J.O.B.H.J.B.B.W.e.al. A revised nomenclature for allergy An EAACI position statement from the EAACI nomenclature task force. Allergy 56, 813-824 (2001).
**10.** Goldsby R.A, K.T.J.O.B.A.K.J. Immunology. W.H. Freeman and Company, (2003).
**11.** Holliday MR, C.E.S.S.B.D.D.R.K.I. Differential induction of cutaneous TNF-alpha and IL-6 by topically applied chemicals. American Journal of Contact Dermatitis 8, 158-164 (1997).
**12.** Smith H.R, B.D.A.a.M.J.P. Irritant dermatitis, irritancy and its role in allergic contact derma. Clinical and Experimental Dermatology 27, 138-146 (2001).
**13.** U.S. Department of Health and Human Services , Food and Drug Administration, and Center for Drug Evaluation and Research (CDER). Guidance for Industry Immunotoxicology Evaluation of Investigational New Drugs. 2002.
**14.** Dean JH, T.L.E.T.R.R.S.D.M.H.D.G.a.S.W.S. ICCVAM evaluation of the murine local lymph node assay. II. Conclusions and recommendations of an indetendent scientific peer review panel. Regulatory Toxicology and Pharmacology 34, 258-273 (2001).
**15.** Dearman R.J, B.D.A.a.K.I. Characterization of Chemical Allergens as a Function of Divergent Cytokine Secretion Profiles Induced in Mice. Toxicology and applied pharmacology 138, 308-316 (1996).
**16.** Dearman RJ, W.E.S.R.K.I. Cytokine fingerprinting of chemical allergens: species comparisons and statistical analyses. Food & Chemical Toxicology 40, 1881-92 (2002).
**17.** Botham P.A. The validation of in vitro methods for skin irritation. Toxicology LEtters 149, 387-90 (2004).
**18.** NOTOX Safety and Environmental Research. Acceptance of in vitro data. 2005.
**19.** Benezra C, S.C.C.P.L.R.H.T.M.H.I. A Systematic Search for Structure-Activity Relationships of Skin Contact Sensitizers. The Journal of Investigative Dermatology 85, 351-356 (1985).
**20.** Ziegler-Heitbrock HW, T.E.F.A.H.V.W.A.R.G. Establishment of a human cell line (Mono Mac &) with characteristics of mature monocytes. Int J Cancer 41 (3), 456-61 (1988).
**21.** Ziegler-Heitbrock H.W et. al. Establishment of a human cell line (Mono Mac 6) with characteristic of mature monocytes. International Journal of cancer 41, 456-461 (1988).
**22.** Läkemedelsindustriföreningen, LIF. 2005.
**23.** Chin K-C and Cresswell P. Viperin (cig5), an IFN-inducible antiviral protein directly induced by human cytomegalovirus. PNAS 98, 15125-130 (2001).
**24.** de Veer M.J, S.H.e.al. IFI60/ISG60/IFIT4, a new member of the human IFI54/IFIT2 family of interferon-stimulated genes. Genomics 54, 267-277 (1998).
**25.** Deblandre G.A, M.O.P.et.al. Expression cloning of an interferon-inducible 17-kDa membrane protein implicated in the control of cell growth. The Journal of Biological Chemistry 270, 23860-66 (1995).
**26.** Haller O and Kochs G. Interferon-induced Mx proteins: Dynamin-like GTPases with antiviral activity. Traffic 3, 710-717 (2002).
**27.** Tissot C and Mechti N. Molecular cloning of an new interferon-induced factor that represses human immunodeficiency virus type 1 long terminal repeat expression. The Journal of Biological Chemistry 270, 14891-898 (1995).
**28.** Henkel G and Krebs B. Metallothioneins: Zinc, Cadmium, Mercury, and Copper Thiolates and Selenolates Mimicking Protein Acrive Site Features - Structural Aspects and Biological Implications. Chem. Rev. 104, 801-824 (2004).
**29.** Richards R, H.A.a.K.M. Structural and functional analysis of the human metallothionein-IA gene: Differential induction by metal ions and glucocortioids. Cell 37, 263-272 (1984).
**30.** Lewis C.E, M.J.O.D. The Macrophage. Oxford University Press, Oxford University, United States (1992).
**31.** Bayard PJ, B.T.J.M. Drug hypersensitivity reactions and human immunodeficiency virus desease. Journal of Acquired Immune Deficiency Syndromes 5, 1237-57 (1992).
**32.** Pirmohamed M, D.J.et.al. The danger hypothesis - potential role in idiosyncratic drug reactions. Toxicology 181-182, 55-63 (2002).
**33.** Facchetti F, V.W.M.D.C.M. The plasmacytoid monocyte/interferon producing cells. Virchows Arch 443, 703-717 (2003).
**34.** Maret W and Vallee B.L. 'Thiolate ligands in metallothioneins confer redox activity on zinc clusters. Preceedings in the National Academy of Sciences of the United States of America 95, 3478-82 (1998).

## Claims

1. A process for in vitro evaluation of a potentially allergenic or tissue irritating substance, **characterised in that** test cells are cultivated in the presence of the substance, and the presence of up regulated genes chosen from MT1G, MT1B;MT1A, MT1E, MT1F, MT1H or expression products from them are measured.

2. The process according to claim 1, **characterised in that** the expression of one or more of genes MT1G, MT1B;MT1A, MT1E, MT1F, MT1H indicates Type IV allergy.

3. The process according to any of claims 1-2, **characterised in that** RNA, DNA, amino acids, peptides or proteins are measured.

4. The process according to any of claims 1-3, **characterised in that** the test cells are chosen from primary blood cells; whole blood, peripheral blood, lymphocytes, monocytes, and cells cultivated in vitro derived from blood cells or cell lines cultivated in vitro.

5. The process according to any of claims 1-4, **characterised in that** the highest concentration of the substance being non toxic to the cells is serial diluted.

6. The process according to any of claims 1 - 5, **characterised in that** cell proliferation is measured.

7. Use of the expression products from one or more of the following genes MT1G, MT1B;MT1A, MT1E, MT1F, MT1H for in vitro analysis of allergy or tissue irritation.

8. Use of a nucleic acid molecule comprising at least 5, preferably at least 10 such as 3-50, 5-40 or 10-30 nucleotides chosen from RNA or DNA complementary to the RNA or DNA corresponding to any of the following genes MT1G, MT1B;MT1A, MT1E, MT1F, MT1H as a probe for in vitro analysis of allergy or tissue irritation.

9. A reagent kit comprising one or more probes, **characterised in that** the probes recognize products produced during the expression of any of MT1G, MT1B;MT1A, MT1E, MT1F, MT1H.

10. The reagent kit according to claim 9, further also comprising test cells.
